(19) Europäisches Patentamt
European Patent Office
Office européen des brevets

(11) EP 0 507 154 B1

(12) **EUROPÄISCHE PATENTSCHRIFT**

(45) Veröffentlichungstag und Bekanntmachung des Hinweises auf die Patenterteilung: **08.05.1996 Patentblatt 1996/19**

(51) Int. Cl.$^6$: **C07C 233/33**, C07C 233/25, C07D 277/50, G01N 33/98

(21) Anmeldenummer: **92104755.1**

(22) Anmeldetag: **19.03.1992**

(54) **Verfahren zur Bestimmung von Alkoholen und Verwendung einer Vorrichtung zur Alkoholbestimmung**

Process for the determination of alcohols and use of a device for determining alcohols

Procédé pour la détermination des alcools et utilisation d'un appareil pour la détermination des alcools

(84) Benannte Vertragsstaaten:
**AT BE CH DE DK ES FR GB IT LI LU NL SE**

(30) Priorität: **21.03.1991 CH 875/91**

(43) Veröffentlichungstag der Anmeldung: **07.10.1992 Patentblatt 1992/41**

(73) Patentinhaber: **Willi Möller AG**
**CH-8050 Zürich (CH)**

(72) Erfinder:
- **Simon, Wilhelm, Prof. Dr.**
  **8006 Zürich (CH)**
- **Seiler, Kurt, Dr.**
  **8409 Winterthur (CH)**

(74) Vertreter: **Blum, Rudolf Emil Ernst et al**
**c/o E. Blum & Co**
**Patentanwälte**
**Vorderberg 11**
**CH-8044 Zürich (CH)**

(56) Entgegenhaltungen:
**EP-A- 0 281 829**

- **ANALYTICA CHIMICA ACTA, Band 233, Nr. 1, 1. Juni 1990, Seiten 41-47, Amsterdam, NL; C. BEHRINGER et al.: "Anion selectivities of trifluoroacetophehone derivatives as neutral ionophores in solvent-polymeric membranes"**
- **ANALYTICAL CHEMISTRY, Band 60, Nr. 14, 15. juli 1988, Seiten 1377-1380, Washington, US; F.L. DICKERT et al.: "Substituted 3,3-diphenylphthalides as optochemical sensors for polar solvent vapors"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 507 154 B1

Printed by Rank Xerox (UK) Business Services
2.11.5/3.4

## Beschreibung

Die vorliegende Erfindung betrifft ein Verfahren zur Bestimmung von aliphatischen oder cycloaliphatischen Alkoholen, bei dem diese mit bestimmten Ketoverbindungen in Berührung gebracht werden, wobei sich bei Kontakt des Alkohols mit der Ketoverbindung reversibel ein Hemiacetal bildet. Die Hemiacetale unterscheiden sich von den Ketoverbindungen, aus denen sie gebildet wurden, bezüglich ihrer Lichtabsorption im ultravioletten, sichtbaren oder infraroten Lichtbereich, oder es entsteht bei der Bildung des Hemiacetals eine Fluoreszenz oder Lumineszenz oder eine solche wird gelöscht. Somit ermöglicht dieses Verfahren die optische Bestimmung von Alkoholen in Proben.

Entsprechende Vorrichtungen zur Durchführung des Bestimmungsverfahrens enthalten als optischen Indikator die entsprechende Ketoverbindung und die Aenderung der optischen Eigenschaft des Indikators, beispielsweise eine Aenderung des Absorptionsspektrums entspricht in ihrer Grössenordnung der Alkoholkonzentration in der Probe. Diese Vorrichtungen können somit zur qualitativen, quantitativen oder halb-quantitativen Bestimmung der Alkoholkonzentration in Proben herangezogen werden.

<u>BESCHREIBUNG DES STANDES DER TECHNIK</u>

Eine rasche Bestimmungsmöglichkeit von Alkoholen, insbesondere Aethanol in biologischen Flüssigkeiten, ist von grosser Bedeutung und eine derartige Bestimmungsmöglichkeit ist auch für viele biotechnologische Verfahren wichtig. Es sei in diesem Zusammenhang beispielsweise auf die Veröffentlichungen von A. Wiseman, Trends Anal. Chem. 7 (1988) 5 und J. Ruz, A.Fernandez M.D., Luquede Castro und M.J. Yalcacel, Pharm. Biomed. Anal., 4 (1986) 559 verwiesen.

Bei der Bestimmung von Aethanol wird dieses im allgemeinen enzymatisch umgewandelt. Erfolgt diese Umwandlung mit Hilfe von Alkoholoxydasen, dann ist die quantitative oder halbquantitative Bestimmung des Aethanols anhand des Verbrauches des Sauerstoffes in dieser enzymatischen Reaktion möglich. Es sei in diesem Zusammenhang auf die Veröffentlichungen von K. P. Völkl, N. Opitz und W.D. Lübbers, Fresenius Z. Anal. Chem., 301 (1980) 162 und O.S. Wolfbeis und H.E. Posch, Fresenius Z. Anal. Chem., 332 (1988) 255 verwiesen.

Erfolgt die enzymatische Umwandlung von Aethanol mit Hilfe von Dehydrogenasen, dann wird die Alkoholkonzentration im allgemeinen anhand der Geschwindigkeit der Bildung von Nebenprodukten optisch festgestellt, beispielsweise anhand der Geschwindigkeit des Uebergangs von Nicotinamid-Adenin-Dinucleotid (abgekürzt als NAD) von seiner oxydierten Form in die reduzierte Form, abgekürzt als NADH. Von der Vielzahl von Veröffentlichungen, die sich mit derartigen enzymatischen Methoden der Aethanolbestimmung befassen, sei beispielsweise auf die aus jüngster Zeit stammende Veröffentlichung von S.M. Gautier, L.J. Blum und P.R. Coulet, J. Biolumin. Chemilumin., 5 (1990) 57, verwiesen.

Bestimmungen, die auf Basis von enzymatischen Reaktionen beruhen, sind jedoch im allgemeinen zeitaufwendig, relativ kompliziert durchführbar, und der Gefahr von Fehlern aufgrund einer Zersetzung bei der Lagerung oder Vergiftung der Enzymkomponente unterworfen.

Sensoren auf Basis von Triphenylmethan-Farbstoffen, die ihre optischen Eigenschaften ändern, sobald sie mit Dämpfen von polaren Lösungsmitteln, einschliesslich Aethanol, in Berührung kommen, wurden bereits zur Durchführung von kontinuerlichen Analysen eingesetzt. Es sei in diesem Zusammenhang auf die Veröffentlichung von H.E. Posch, O.S. Wolfbeis und J. Pusterhofer, Talanta, 35 (1988) 89 verwiesen. Auch in der Veröffentlichung von F.L. Diekert, E.H. Lehmann, S.K. Schreiner, H. Kimmel und G.R. Mages, Anal. Chem. 60 (1988), Seiten 1377 bis 1388, werden entsprechende Sensoren für Dämpfe von polaren Lösungsmitteln beschrieben, in welchen der in dem Sensor enthaltene Farbstoff ein Lacton eines triphenylsubstituierten Methanols ist, in welchem an jede der drei Phenylgruppen der Triphenylmethangruppierung in der para-Stellung derselben eine Dimethylaminogruppe gebunden ist. Eine der drei Phenylgruppen ist ausserdem in der ortho-Stellung derselben mit einer Carbonsäuregruppierung substituiert, die zusammen mit der Hydroxygruppierung des entsprechenden Methanolderivates den fünfgliedrigen Lactonring bildet. Dieser Lactonring wird jedoch leicht gespalten, wobei sich die entsprechenden gefärbten Triphenylcarbeniumionen bilden, die eine charakteristische Absorptionsbande bei 610 nm aufweisen. Die entsprechenden Farbstoffe können als chemische Sensoren für Aethanol und andere polare Lösungsmittel eingesetzt werden.

Ein wesentlicher Nachteil der in dieser Veröffentlichung beschriebenen Sensoren besteht darin, dass die entsprechenden Testresultate durch die Feuchtigkeit der Umgebung verfälscht werden (siehe die Fig. 4 auf Seite 1379, linke Spalte dieser Veröffentlichung) und dass ferner die Sensoren keine Selektivität für Alkohole besitzen, sondern dass sie auch auf andere polare Lösungsmittel, wie Aceton und Essigsäureäthylester ansprechen.

Weit verbreitet sind ferner Geräte zur halbquantitativen Bestimmung der Blutalkohol-Konzentration über die ausgeatmete Atemluft von Versuchspersonen, die unter dem Markennamen "Alcotest" vertrieben werden. Diese Vorrichtungen basieren auf der Reduktion von Chromatschwefelsäure, die sich auf einem inerten Trägermaterial befindet und gelb gefärbt ist, durch das Aethanol zu grüngefärbten Verbindungen des dreiwertigen Chroms. Auch diese Farbreaktion ist natürlich nicht für Alkohole spezifisch, sondern spricht auf jede beliebige Substanz an, die von der Chromatschwefelsäure unter den angegebenen Bedingungen oxydierbar ist.

In der am 14. September 1988 veröffentlichten europäischen Patentveröffentlichung Nr. 0 281 829 von Willi Möller AG werden ferner Ketoverbindungen beschrieben, die selektiv mit Anionen von Oxasäuren in Wechselwirkung treten und mit diesen Addukte bilden. Beispiele für Anionen von Oxasäuren, die zu derartigen Adduktbildungen geeignet sind, sind Anionen von Carbonsäuren, Hydroxycarbonsäuren, Ketocarbonsäuren, Aminocarbonsäuren und Anionen von Peptiden, sowie Anionen anorganischer Oxasäuren, wie zum Beispiel Carbonatanionen und Phosphatanionen. Die entsprechenden Ketoverbindungen weisen mindestens einen stark elektronenanziehenden Substituenten und ein $\pi$-Elektronensystem auf, das sich in Konjugation zu der Ketogruppe befindet. Die bevorzugten, mit den Oxasäuren Addukte bildenden Ketoverbindungen sind Monoketone der folgenden Formel A

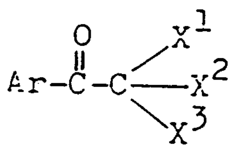

A

in welcher
Ar ein aromatischer oder heteroaromatischer Rest ist und
$X^1$, $X^2$ und $X^3$ unabhängig voneinander für Wasserstoffatome, Alkylgruppen, Alkenylgruppen, Alkinylgruppen, Fluoratome, Chloratome, Bromatome oder Nitrogruppen stehen, unter der Voraussetzung, dass jedoch mindestens einer dieser Substituenten ein stark elektronenanziehender Substituent, ausgewählt aus der Gruppe Fluoratome, Chloratome, Bromatome oder Nitrogruppen ist.

Ketoverbindungen, die der oben angegebenen Formel A entsprechen, in welchen
Ar ein Phenylrest ist, der in der para-Stellung zur Ketogruppe mit einem Substituenten substituiert ist, der aus der Gruppe von Substituenten ausgewählt ist, welche Dialkylaminogruppen, acylierte Monoalkylaminogruppen, Carbonsäureestergruppen, Carbonsäureamidgruppen, Alkylgruppen, Aethergruppen, Thioäthergruppen und Sulfoxidgruppen umfassen, und in denen ausserdem
$X^1$, $X^2$ und $X^3$ Fluoratome sind,
werden in der Veröffentlichung von Christoph Behringer, Beatrice Lehmann, Jean-Pierre Haug, Kurt Seiler, Werner E. Morf, Karel Hartman und Wilhelm Simon in Analytica Chimica Acta, Band 233, 1990, Seiten 41-47 beschrieben. In dieser Veröffentlichung wird die Selektivität der entsprechenden Trifluoromethylketone für Carbonatanionen, im Vergleich zu Chloridanionen, bestimmt, und auch die Hydratbildung dieser Verbindungen bei Einwirkung von Feuchtigkeit wurde untersucht.

Ziel der vorliegenden Erfindung war es, neue Sensoren zu entwickeln, die eine höhere Spezifität für aliphatische und cycloaliphatische Alkohole aufweisen, als die aus dem Stande der Technik bekannten Sensoren auf Basis von Triphenylmethanfarbstoffen. Die entsprechenden Sensoren sollten mit den genannten Alkoholen Reaktionsprodukte liefern, die sich von dem zu deren Herstellung eingesetzten Ausgangsmaterial in der Lichtabsorption im ultravioletten, sichtbaren oder infraroten Bereich der Wellenlängen unterscheiden, oder bei denen bei Kontakt mit dem zu bestimmenden Alkohol eine Fluoreszenz oder Lumineszenz hervorgerufen oder gelöscht wird. Des weiteren sollten die zu verwendenden Sensoren nicht diejenigen Nachteile aufweisen, die bei Systemen zur enzymatischen Bestimmung von Alkoholen anzutreffen sind.

Ueberraschenderweise zeigte es sich, dass ein Verfahren zur Bestimmung von Alkoholen unter Verwendung von bestimmten Ketoverbindungen durchgeführt werden kann, wobei diese Ketoverbindungen entweder neue Ketoverbindungen darstellen oder aus der Literatur bekannte Ketoverbindungen sind, einschliesslich derjenigen, welche die weiter vorne angegebene Formel A aufweisen. Bei diesen Arbeiten zeigte es sich ferner, dass die Ketoverbindungen dann, wenn sie mit dem zu bestimmenden Alkohol in Berührung kommen, reversibel in die entsprechenden Hemiacetale umgewandelt werden und es zeigte sich ferner, dass sich die Hemiacetale von den Ketoverbindungen bezüglich ihrer Lichtabsorption im ultravioletten, im sichtbaren oder im infraroten Bereich der Wellenlängen unterscheiden, oder dass bei der Bildung der Hemiacetale aus den Ketoverbindungen eine Fluoreszenz oder Lumineszenz gebildet oder gelöscht wird.

<u>BESCHREIBUNG DER ERFINDUNG</u>

Ein Gegenstand der vorliegenden Erfindung ist ein Verfahren zur Bestimmung von Alkoholen der Formel

$$R'-OH$$

in welcher

R' ein aliphatischer oder cycloaliphatischer Rest ist. Dieses Verfahren ist dadurch gekennzeichnet, dass man die Alkohole mit einer Ketoverbindung der Formel I

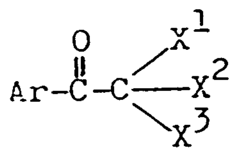

I

in Berührung bringt, in welcher

Ar ein unsubstituierter oder substituierter einkerniger oder mehrkerniger, aromatischer Rest oder ein unsubstituierter oder substituierter ein- oder mehrkerniger heterocyclischer Rest aromatischen Charakters ist und die Substituenten unpolare Substituenten, Substituenten mit schwach polaren Eigenschaften, Substituenten mit basischen Eigenschaften und/oder chromophore Gruppen, beziehungsweise Gruppen, die eine Fluoreszenz oder Lumineszenz induzieren, sind, und

$X^1$, $X^2$ und $X^3$ unabhängig voneinander Wasserstoffatome, Alkylgruppen, Alkenylgruppen, Alkinylgruppen, Fluoratome, Chloratome, Bromatome oder Nitrogruppen bedeuten, unter der Voraussetzung, dass jedoch mindestens einer der Substituenten $X^1$, $X^2$ und $X^3$ ein stark elektronenanziehender Substituent, ausgewählt aus der Gruppe Fluoratome, Chloratome, Bromatome oder Nitrogruppen ist,

wobei sich bei dem Kontakt des Alkohols mit der Ketoverbindung der Formel I reversibel ein Hemiacetal der Formel II

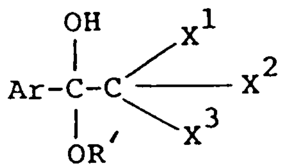

II

bildet und wobei sich die Ketoverbindung der Formel I von dem Hemiacetal bezüglich der Lichtabsorption im ultravioletten, sichtbaren oder infraroten Lichtbereich unterscheidet oder dass bei der Bildung des Hemiacetales eine Fluoreszenz oder Lumineszenz entsteht oder gelöscht wird.

Mit Hilfe des erfindungsgemässen Verfahrens kann man eine qualitative, quantitative oder halb-quantitative Bestimmung der Alkohole in flüssigen oder gasförmigen Proben durchführen, beispielsweise in alkoholischen Getränken oder flüssigen oder gasförmigen Proben biologischen Ursprungs, beispielsweise Harn, Blut, Blutserum, Blutplasma oder Atemluft, indem man diese Proben mit einem optischen Sensor in Berührung bringt, der als optischen Indikator eine Ketoverbindung der Formel I enthält.

Entsprechende Vorrichtungen zur Durchführung des erfindungsgemässen Verfahrens sind zur optischen Bestimmung der Alkohole verwendbar und sie enthalten als optischen Indikator eine Ketoverbindung der Formel I, die bei Kontakt mit den zu bestimmenden Alkoholen der Formel

R'OH,

in welchen R' ein aliphatischer oder cycloaliphatischer Rest ist,

einer Reaktion unter Hemiacetalbildung unterliegt, wobei sich die Ketoverbindung der Formel I von dem entsprechenden Hemiacetal der Formel II bezüglich der Lichtabsorption im ultravioletten, sichtbaren oder infraroten Lichtbereich unterscheidet, oder wobei durch die Bildung des Hemiacetales eine Fluoreszenz oder Lumineszenz entsteht oder gelöscht wird.

Ein weiterer Gegenstand der vorliegenden Erfindung ist die Verwendung einer Vorrichtung die eine Ketoverbindung der Formel I enthält zur qualitativen, quantitativen oder halb-quantitativen Bestimmung von Alkoholen der Formel

R'OH

in welchen R' ein aliphatischer oder cycloaliphatischer Rest ist, wobei diese Verwendung dadurch gekennzeichnet ist, dass man flüssige oder gasförmige Proben, in welchen der Alkoholgehalt bestimmt werden soll, mit der Vorrichtung in

Berührung bringt, wobei die in der Vorrichtung als Indikator anwesende Ketoverbindung der Formel I mit dem Alkohol unter Hemiacetalbildung reagiert und dabei eine Aenderung des Absorptionsspektrums hervorgerufen wird, die in ihrer Grössenordnung der Alkoholkonzentration entspricht.

In bevorzugten Ketoverbindungen der Formel I, die zur Durchführung des erfindungsgemässen Verfahrens herangezogen werden, ist Ar ein Benzolkern, ein Naphthalin-, Phenanthren- oder Anthracengerüst oder ein ein- oder mehrkerniger heterocyclischer Rest aus der Gruppe Thiophen, Furan, Benzofuran, Benzothiophen, und vorzugsweise ein ein- oder mehrkerniger heterocyclischer Rest aromatischen Charakters, der mindestens ein Stickstoffatom als Heteroatom aufweist, vorzugsweise Pyrrol, Diazol, Triazol, Pyridin, 1,2-Diazin, 1,3-Diazin, 1,4-Diazin (Pyrazin), 1,3,5-Triazin, 1,2,4-Triazin oder 1,2,3-Triazin, Tetrazin, Thiazol, Monoazanaphthaline, wie 1-Azanaphthalin (Chinolin) und 2-Azanaphthalin (Isochinolin), Diazanaphthaline, Triazanaphthaline, Indol, Carbazol, Monoazaanthracene, Monoazaphenanthrene, beispielsweise Acridin und Phenanthridin, Diazaanthracene und Diazaphenanthrene, beispielsweise Phenanthrolin, sowie Triaza- und Tetraazaanthracene und -phenanthrene.

In den Ketoverbindungen der Formel I, beziehungsweise in den Hemiacetalen der Formel II, können die aromatischen, beziehungsweise heteroaromatischen Reste Ar unsubstituiert sein oder sie können einen oder mehrere Substituenten tragen, die unpolare Substituenten, Substituenten mit schwach polaren Eigenschaften oder Substituenten mit basischen Eigenschaften sind und/oder die chromophore Gruppen darstellen oder solche Gruppen, die in der Lage sind, eine Fluoreszenz oder Lumineszenz zu induzieren.

Im allgemeinen sind an den aromatischen, beziehungsweise heteroaromatischen Rest Ar der Ketoverbindungen der Formel I, beziehungsweise an die oben genannten bevorzugten aromatischen, beziehungsweise heteroaromatischen Reste Ar dieser Verbindungen 0 bis 5 Substituenten gebunden, vorzugsweise 1 bis 4 Substituenten, die unpolare Eigenschaften oder nur leicht polare Eigenschaften aufweisen oder die basische Eigenschaften besitzen.

Als Beispiele für Substituenten mit unpolaren oder nur schwach polaren Eigenschaften seien die folgenden genannt:

Halogenatome, Nitrogruppen, gegebenenfalls substituierte Alkylgruppen, gegebenenfalls substituierte Alkenylgruppen mit ein oder mehr Kohlenstoff-Kohlanstoff-Doppelbindungen, gegebenenfalls substituierte Alkinylgruppen, gegebenenfalls substituierte Cycloalkylgruppen, gegebenenfalls substituierte Arylgruppen und gegebenenfalls substituierte heterocyclische Reste, wobei die in diesen Gruppierungen allfällig anwesenden Substituenten selbst wieder unpolare Substituenten, Substituenten mit nur wenig polaren Eigenschaften oder Substituenten mit basischen Eigenschaften sind. Als weitere Beispiele für unpolare oder wenig polare Substituenten, die an den aromatischen oder heteroaromatischen Rest Ar gebunden sein können, seien Aether- oder Hydroxygruppen der Formel

$$-O-R^1 ,$$

sowie Schwefel enthaltende Gruppen der Formel

$$-S-R^1, -SO-R^1 \text{ und } -SO_2-R^1$$

genannt, wobei in diesen Gruppierungen $R^1$ für Wasserstoff oder gegebenenfalls substituierte Alkylgruppen, gegebenenfalls substituierte Alkenylgruppen, gegebenenfalls substituierte Cycloalkylgruppen, gegebenenfalls substituierte Arylgruppen und gegebenenfalls substituierte Heterocyclen steht, oder die entsprechenden unpolaren oder wenig polaren Substituenten des aromatischen oder heteroaromatischen Restes Ar sind, Nitrilgruppen, Carbonsäureestergruppen, Carbonsäureamidgruppen, Sulfonsäureestergruppen, Sulfonsäureamidgruppen oder veresterte Hydroxygruppen.

Eine bevorzugte Gruppe an Substituenten, die an die aromatischen oder heteroaromatischen Reste Ar der Ketoverbindungen der Formel I gebunden sein können, sind Substituenten mit basischen Eigenschaften, und von diesen Substituenten sind wieder primäre, sekundäre oder tertiäre Aminogruppen oder acylierte Aminogruppen bevorzugt, also Gruppierungen, welche der folgenden Formel

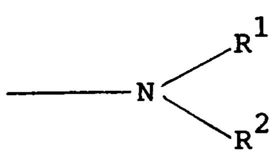

entsprechen, wobei in dieser Gruppierung

$R^1$ und $R^2$ unabhängig voneinander Wasserstoffatome, gegebenenfalls substituierte Alkylreste, gegebenenfalls substituierte Alkenylreste, gegebenenfalls substituierte Alkinylreste, gegebenenfalls substituierte Cycloalkylreste, gegebenenfalls substituierte Arylreste, gegebenenfalls substituierte heterocyclische Reste oder Acylreste der Formel

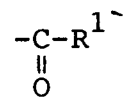

oder der Formel

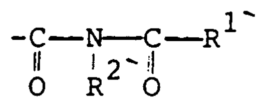

darstellen, wobei in diesen Acylresten

$R^{1`}$ und $R^{2`}$ unabhängig voneinander Wasserstoffatome, gegebenenfalls substituierte Alkylreste, gegebenenfalls substituierte Alkenylreste, gegebenenfalls substituierte Alkinylreste, gegebenenfalls substituierte Cycloalkylreste, gegebenenfalls substituierte Arylreste und gegebenenfalls substituierte heterocyclische Reste darstellen, oder

$R^1$ und $R^2$ bilden zusammen mit dem Stickstoffatom, an welches sie gebunden sind, einen heterocyclischen Rest, der in seiner Ringstruktur frei von weiteren Heteroatomen sein kann, oder allenfalls noch zusätzliche Heteroatome aufweisen kann, vorzugsweise solche, die Sauerstoff, Schwefel oder Stickstoff sind, und wobei die entsprechenden heterocyclischen Reste unsubstituiert oder mit solchen Substituenten substituiert sein können, die unpolare, wenig polare oder basische Eigenschaften aufweisen.

Beliebige Kombinationen der Substituenten sind ebenfalls möglich, wie zum Beispiel arylsubstituierte Alkylgruppen, über Aethergruppierungen gebundene Alkylgruppen, die mit primären, sekundären oder tertiären Aminogruppen oder Acylaminogruppen substituiert sind und ähnliche Substituenten.

In einer bevorzugten Gruppe der Ketoyerbindungen der Formel I, die zur Durchfuhrung des erfindungsgemässen Verfahrens herangezogen werden, sind ferner an den aromatischen, beziehungsweise heteroaromatischen Rest Ar chromophore Gruppen gebunden oder Gruppen, die eine Fluoreszenz oder Lumineszenz induzieren, wie beispielsweise Azogruppen, über Azogruppen gebundene aromatische oder heteroaromatische Reste oder Systeme mit zwei oder mehr konjugierten Doppelbindungen oder konjugierten Dreifachbindungen oder Konjugationen aus Doppelbindungen, Dreifachbindungen und aromatischen Systemen oder heteroaromatischen Systemen.

Als bevorzugtes Beispiel für Ketoverbindungen der Formel I, die eine chromophore Gruppe aufweisen, seien die entsprechenden Azoketoverbindungen der Formel III

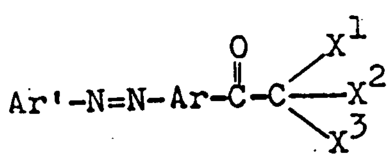

III

genannt,

in welchen

Ar und Ar' unabhängig voneinander substituierte oder unsubstituierte, ein- oder mehrkernige, aromatische oder substituierte oder unsubstituierte, ein- oder mehrkernige heterocyclische Reste aromatischen Charakters bedeuten und

$X^1$, $X^2$ und $X^3$ unabhängig voneinander Wasserstoffatome, Alkylgruppen, Alkenylgruppen, Alkinylgruppen, Fluoratome, Chloratome, Bromatome, oder Nitrogruppen sind, unter der Voraussetzung, dass jedoch mindestens einer der Substituenten $X^1$, $X^2$ und $X^3$ ein stark elektronenanziehender Substituent, ausgewählt aus der Gruppe Fluoratome, Chloratome, Bromatome oder Nitrogruppen ist.

Wenn bei der Durchführung des erfindungsgemässen Verfahrens die Azoketoverbindungen der Formel III mit den zu bestimmenden Alkoholen der Formel

R'OH

in Berührung kommen, dann bildet sich aus dieser ein entsprechendes Azo-hemiacetal der Formel IV

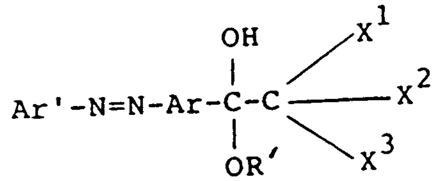

IV

In den zur Durchführung des erfindungsgemässen Verfahrens einsetzbaren Azoketoverbindungen der Formel III und in den aus diesen bei Kontakt mit dem Alkohol gebildeten Azo-hemiacetalen der Formel IV ist der Rest Ar und auch der Rest Ar' vorzugsweise ein Benzolkern, ein Naphthalin-, Phenanthren oder Anthracengerüst oder ein ein- oder mehrkerniger heterocyclischer Rest derjenigen Gruppe, die weiter vorne im Zusammenhang mit den Ketoverbindungen der Formel I und den Hemiacetalen der Formel II als bevorzugte heterocyclische Reste aufgezählt wurde.

In den Azo-hemiacetalen der Formel IV und in den Azoketoverbindungen der Formel III, aus denen diese gebildet wurden, können die beiden Reste Ar und Ar' miteinander gleiche oder voneinander verschiedene ein- oder mehrkernige aromatische Reste darstellen oder sowohl der Rest Ar als auch der Rest Ar' kann ein ein- oder mehrkerniger heterocyclischer Rest aromatischen Charakters, insbesondere einer aus der weiter vorne angegebenen bevorzugten Gruppe an heterocyclischen Resten sein.

Es zeigte sich jedoch, dass bezüglich der Aenderung der optischen Eigenschaften bei der Bildung des Hemiacetales dann besonders vorteilhafte Eigenschaften erreicht werden, wenn in den Azoketoverbindungen der Formel III einer der beiden Reste Ar und Ar' ein ein oder mehrkerniger aromatischer Rest ist und der andere dieser beiden Reste einen ein oder mehrkernigen heterocyclischen Rest aromatischen Charakters darstellt. Von diesen Verbindungen sind wieder diejenigen bevorzugt, in welchen der Rest Ar ein aromatischer Rest, vorzugsweise ein Benzolkern, und der Rest Ar' ein heteroaromatischer Rest ist.

Wenn in den Azoketoverbindungen der Formel III der Rest Ar ein Benzolkern ist, dann ist in diesem die Azogruppe vorzugsweise in para-Stellung zu der Ketogruppe angeordnet, weil dadurch optimale Konjugationsmöglichkeiten zwischen der Carbonylgruppe über den aromatischen Kern Ar, die Azogruppe zu dem System Ar', das ein aromatisches oder heteroaromatisches System sein kann, gegeben sind.

Die durchgeführten Untersuchungen zeigten ferner, dass solche Ketoazoverbindungen der Formel III besonders vorteilhafte Eigenschaften aufweisen, in welchen der Rest Ar ein aromatischer Rest und der Rest Ar' ein stickstoffenthaltender, schwefelenthaltender und speziell bevorzugt, sowohl Stickstoff als auch Schwefel enthaltender hetero-aromatischer Rest ist, beispielsweise ein Thiazol.

Als bevorzugte Beispiele für Azo-hemiacetale der Formel IV seien die entsprechenden Thiazolderivate der folgenden Formel

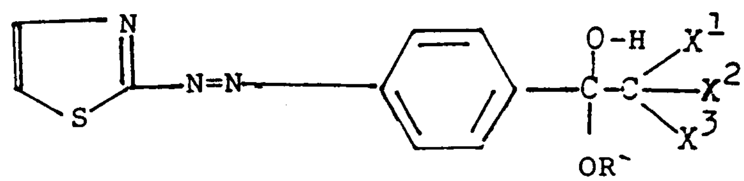

genannt.

In diesen Thiazolderivaten können gegebenenfalls sowohl im Benzolkern als auch im Thiazolring noch weitere Substituenten vorhanden sein, wobei auch hier Substituenten aus der weiter vorne angegebenen Klasse von Substituenten speziell bevorzugt sind.

Bei den beim erfindungsgemässen Verfahren gebildeten Hemiacetalen, bzw. Azohemiacetalen der Formeln II und IV und den Ketoausgangsmaterialien der Formeln I und III ist es wesentlich, dass mindestens einer der Reste $X^1$, $X^2$ und $X^3$, vorzugsweise zwei der genannten Substituenten, und speziell bevorzugt, alle drei der genannten Substituenten, stark elektronenanziehende Substituenten sind, die aus der Gruppe ausgewählt sind, welche Fluoratome, Chloratome, Bromatome oder Nitrogruppen umfassen. In speziell bevorzugten derartigen Verbindungen sind sämtliche der genannten Reste Fluoratome und/oder Chloratome, und speziell bevorzugt, sind entsprechende Ketoverbindungen der Formel I, beziehungsweise III, beziehungsweise Hemiacetale der Formel II, Bzw IV in welchen die Gruppierung der Formel

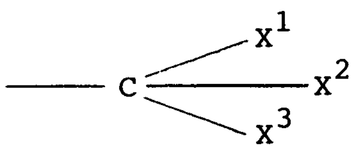

eine Trifluormethylgruppe ist.

Vorzugsweise wird die Bestimmung der Alkohole nach dem erfindungsgemässen Verfahren so durchgeführt, dass sich die Ketoverbindung der Formel I auf einem oder in einem Trägermaterial befindet, vorzugsweise einem Polymermaterial und dass die Umsetzung der Ketoverbindung der Formel I zu dem Hemiacetal der Formel II auf diesem, beziehungsweise in diesem Trägermaterial stattfindet.

In diesem Falle kann also die auf dem Trägermaterial befindliche Ketoverbindung der Formel I als optischer Indikator für die Anwesenheit der entsprechenden Alkohole dienen, weil sich bei der Ueberführung der Ketoverbindung der Formel I in das entsprechende neue Hemiacetal der Formel II die optische Eigenschaft des entsprechenden Indikators verändert.

Die Vorrichtung zur optischen Bestimmung von Alkoholen der Formel

R'OH ,

in welchen

R' ein aliphatischer oder cycloaliphatischer Rest ist, nach dem erfindungsgemässen Verfahren weist vorzugsweise einen optischen Sensor auf, der seine Lichtabsorption im ultravioletten, sichtbaren oder infraroten Lichtbereich ändert, oder bei dem eine Fluoreszenz oder Lumineszenz entsteht oder gelöscht wird, sobald dieser optische Sensor mit einem Alkohol in Berührung kommt, wobei der Sensor als optischen Indikator der angegebenen Art eine Ketoverbindung der Formel I

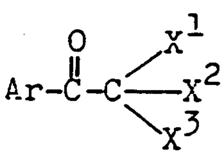

I

enthält, die sich bei Kontakt dieser Ketoverbindung mit dem zu bestimmenden Alkohol in das Hemiacetal der Formel II

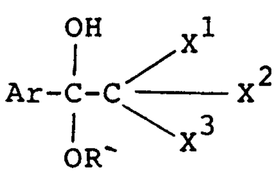

II

umwandelt, das sich bezüglich der angeführten optischen Eigenschaft von der Ketoverbindung der Formel I unterscheidet, wobei Ar, $X^1$, $X^2$ und $X^3$ die weiter vorne angegebene Bedeutung aufweisen und wobei in den entsprechenden Ketoverbindungen der Formel I vorzugsweise sämtliche Reste $X^1$, $X^2$ und $X^3$ Fluoratome sind.

Bei der Verwendung dieser Vorrichtung zur qualitativen, quantitativen oder halbquantitativen Bestimmung von Alkoholen in flüssigen oder gasförmigen Proben bringt man die flüssige oder gasförmige Probe, beispielsweise ein alkoholischen Getränk, eine flüssige oder gasförmige Probe biologischen Ursprungs, beispielsweise Harn, Blut oder Atemluft, mit dem optischen Sensor in Berührung.

Bevorzugte Alkohole der Formel R'OH, die in wässrigen oder gasförmigen Medien mit Hilfe des erfindungsgemässen Verfahrens bestimmt werden können, sind solche, in denen der Rest R' einen Alkylrest mit 1 - 10 Kohlenstoffatomen darstellt, insbesondere entsprechende primäre Alkohole mit 1 - 5 Kohlenstoffatomen.

Die Ketoverbindungen der Formel I, beziehungsweise die Azoketoverbindungen der Formel III, befinden sich vorzugsweise in einem oder auf einem Trägermaterial und auch in den erfindungsgemäss verwendeten Vorrichtungen enthält der optische Sensor vorzugsweise ein Trägermaterial, in welchem oder auf welchem sich die Ketoverbindung der Formel I, beispielsweise eine Azoketoverbindung der Formel III befindet.

Bevorzugte derartige Trägermaterialien sind Polymermaterialien, wie beispielsweise Cellulose, Cellulosederivate, siliciumenthaltende Polymermaterialien beispielsweise Siliconkautschuk, Polymermaterialien auf Basis von Polyestern, Polyamiden, Polyäthern oder Homopolymerisate oder Copolymerisate äthylenisch ungesättigter Monomere. Als Beispiele für Homopolymerisate und Copolymerisate äthylenisch ungesättigter Monomere, seien Homopolymerisate und Copolymerisate der folgenden Monomerkomponenten genannt: Styrol, Vinylacetat, Vinylalkohol, Butadien, Aethylen, Propylen und insbesondere halogenierte ungesättigte Monomere, beispielsweise Homopolymerisate oder Copolymerisate von chlorierten oder fluorinierten äthylenisch ungesättigten Monomermaterialien, insbesondere Vinylchlorid oder Vinylidenchlorid.

Gegebenenfalls können die entsprechenden Polymermaterialien, in denen die Ketoverbindungen der Formel I, beispielsweise die Azoketoverbindungen der Formel III, eingebettet sind, ausserdem noch einen Weichmacher enthalten, wobei Weichmacher mit relativ stark lipophilen Eigenschaften im allgemeinen bevorzugt sind.

Als Weichmacher für entsprechende Polymermaterialien, wie zum Beispiel solche auf Basis von Polyvinylchlorid Homopolymerisaten und Copolymerisaten, haben sich Aetherweichmacher und Esterweichmacher als besonders geeignet erwiesen.

Als Aetherweichmacher sind insbesondere solche geeignet, bei denen sowohl ein aromatischer Rest, als auch ein längerkettiger aliphatischer Rest an das Aethersauerstoffatom gebunden ist, wie zum Beispiel der o-Nitrophenyl-octyläther.

Als Beispiele für Esterweichmacher seien solche auf Basis von Dicarbonsäurediestern und Tetracarbonsäuretetraestern genannt, beispielsweise diejenigen, in welchen der esterbildende Alkohol ein längerkettiger aliphatischer Alkohol ist, beispielsweise ein Alkanol mit 4 bis 24, vorzugsweise 5 - 12 Kohlenstoffatomen. Als Beispiele für entsprechende Dicarbonsäurediester seien diejenigen der Adipinsäure und Sebacinsäure, wie zum Beispiel Bis(2- äthylhexyl)-sebacinsäureester erwähnt und als Beispiele für Tetracarbonsäuretetraester solche von Benzophenontetracarbonsäuren und Benzhydroltetracarbonsäuren genannt, die beispielsweise in den U.S. Patentschriften 4 783 496 und 4 857 573 von Simon et al. beschrieben sind.

Seit langem ist ferner bekannt, dass bestimmte Komponenten, insbesondere aus der Gruppe der Dicarbonsäurediamide, die Fähigkeit besitzen, mit Kationen lipophile Komplexe zu bilden und Polymermaterialien, die diese kationselektiven Komponenten und im allgemeinen ausserdem einen Weichmacher enthalten, werden zur elektrometrischen Bestimmung der entsprechenden Kationen in vielen Bereichen eingesetzt, insbesondere auch auf dem klinischen Gebiet.

In analoger Weise können auch mit entsprechenden anionselektiven Komponenten Anionen bestimmt werden.

Wenn der kationselektive lipophile Komplexbildner eine chromophore Gruppe enthält, die zu einer Aenderung der optischen Eigenschaften des Komplexbildners bei der Bildung des Komplexes mit dem entsprechenden Kation führt, oder wenn eine optische Eigenschaft aufgrund einer bei der Komplexbildungsreaktion freigesetzten Substanz, beispielsweise eines Protons, mit Hilfe eines pH-Indikators, feststellbar ist, dann können die entsprechenden Membranen auch zur optischen Bestimmung der Kationkonzentration herangezogen werden, und analoge Ausführungen sind auch für die Bestimmung von Anionen bekannt. In diesen Fällen hat es sich als vorteilhaft erwiesen, wenn in die Polymermembran zusätzlich zu den ionenselektiven Komplexbildnern noch eine Komponente einverleibt wird, die zu einer Coextraktion des Kations mit dem entsprechenden salzbildenden Anion aus der wässrigen Phase in die Phase des Polymermaterials führt, beziehungsweise die zu einem Ionenaustausch eines in dem Polymermaterial enthaltenen Anions gegen ein Anion der Probelösung führt. Typische Beispiele für in dem Polymermaterial enthaltene Substanzen, die zu einem Anionenaustausch gegen Anionen der Probelösung befähigt sind, sind quaternäre Ammoniumsalze mit mindestens einer an das Stickstoffatom gebundenen lipophilen Kette, wie zum Beispiel einer Alkylkette mit 4 - 20 Kohlenstoffatomen.

Obwohl die mit Hilfe des erfindungsgemässen Verfahrens unter Verwendung der Vorrichtungen zu bestimmenden Alkohole eindeutig keine ionischen Substanzen sind, so hat es sich in völlig unerwarteter Weise dennoch gezeigt, dass es vorteilhaft ist, in das Polymermaterial des Sensors ausserdem eine Komponente einzuverleiben, die zu einem Ionenaustausch mit Ionen des wässrigen Systems der flüssigen oder gasförmigen Proben befähigt ist. Als Beispiele für derartige Substanzen, die zu einem Ionenaustausch fähig sind, seien quaternäre Ammoniumsalze genannt, insbesondere solche, bei denen mindestens eine Alkylgruppe mit 4 - 20 Kohlenstoffatomen, an das Stickstoffatom des quaternären Ammoniumsalzes gebunden ist. Beispielsweise sind die Halogenidanionen von entsprechenden quaternären Ammoniumhalogeniden zum Ionenaustausch gegen Ionen der flüssigen oder gasförmigen Proben befähigt, und es zeigte sich, dass bei einem Kontakt mit wässrigen Proben ein Austausch des Halogenidanions dieses quaternären Ammoniumsalzes gegen Hydroxylionen der Probelösung möglich ist. Durch einen solchen Ionenaustausch wird der pH-Wert innerhalb des Polymermaterials in Richtung basisch verschoben, ein Vorgang, der erwünscht ist, wenn man bedenkt, dass die Reaktion einer Ketoverbindung der Formel I mit einem Alkohol der Formel R'OH unter Bildung des Hemiacetals der Formel II unter basischen Bedingungen katalysiert wird.

Somit führen Ketoverbindungen der Formel I, in denen die Substituenten am aromatischen Rest Ar basische Eigenschaften aufweisen, beziehungsweise in denen der Rest Ar ein heterocyclischer Rest mit basischen Eigenschaften, wie zum Beispiel ein Pyridinkern ist, zu einer Katalysierung und damit zu einem raschen Ablauf der Bildung des Hemiacetals. Das gleiche trifft auch für weitere Komponenten zu, die allenfalls in einem solchen Sensor enthalten sein können, wie

die erwähnten quaternären Ammoniumsalze, die dann durch Ionenaustausch bei der Durchführung der Bestimmung eine basische Umgebung gewährleisten.

Im Prinzip wären die aus der Ketoverbindung der Formel I und dem Alkohol der Formel R'OH gebildeten Hemiacetale der Formel II befähigt, mit einem weiteren Molekül des Alkohols der Formel R'OH unter Ausbildung des entsprechenden Vollacetales zu reagieren (also Austausch der OH-Gruppe des Hemiacetales gegen einen weiteren Rest OR' unter Abspaltung von Wasser). Die Bildung des Vollacetales ist jedoch unerwünscht, weil dadurch die Empfindlichkeit der Bestimmungsmethode verringert, beziehungsweise bei quantitativen Bestimmungen das Ergebnis verfälscht wird. Der Uebergang des Hemiacetales in das Vollacetal wird durch saure pH-Werte katalysiert. Auch aus diesem Grunde ist es vorteilhaft, wenn durch die Wahl der Substituenten und/oder des heterocyclischen Restes und/oder die Einverleibung einer weiteren Komponente dafür Sorge getragen wird, dass bei der Durchführung der Bestimmung eine Verschiebung des pH-Bereiches in ein saures Milieu, verhindert wird.

Die Ketoverbindungen der Formel I sind ferner in der Lage, mit Wasser Hydrate zu bilden, die eine ähnliche Struktur besitzen, wie die Hemiacetale der Formel II. Die fraglichen Hydrate entsprechen nämlich der folgenden Formel V

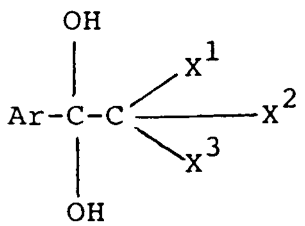

V

Diese Bildung der Hydrate ist ebenfalls eine basisch katalysierte Reaktion. Dennoch ist überraschenderweise mit den erfindungsgemässen Vorrichtungen eine Bestimmung der Alkohole in wässrig alkoholischen Lösungen mit guten Empfindlichkeiten möglich, wobei besonders hohe Empfindlichkeiten dann erzielt werden, wenn die Ketoverbindung der Formel I eingebettet in einem Polymermaterial vorliegt.

Wie bereits erwähnt wurde, ist schon aus der europäischen Patentveröffentlichung 0 281 829 bekannt, dass Ketoverbindungen der Formel A mit Oxasäuren, beispielsweise Carbonsäuren, Addukte bilden. Aus diesem Grunde war zu erwarten, dass die erfindungsgemässen Vorrichtungen nicht zur Bestimmung von Alkohol in solchen Systemen herangezogen werden können, die als weitere Komponente organische Säuren enthalten. Im Gegensatz zu dieser Annahme, zeigte es sich jedoch, dass die erfindungsgemässen Vorrichtungen hervorragend zur Bestimmung von Alkohol in wässrigen Systemen, wie Weinen, geeignet sind, die bekanntlich relativ hohe Gehalte an organischen Säuren aufweisen.

Ein Vergleich der Ketoverbindungen der Formel I mit den Hemiacetalen der Formel II zeigt, dass bei den Ketoverbindungen der Formel I eine Konjugation zwischen der Kohlenstoff-Sauerstoff-Doppelbindung der Ketogruppen und dem aromatischen System Ar des aromatischen, beziehungsweise heteroaromatischen Restes vorliegt, und dass diese Konjugation durch die Bildung des Hemiacetales aufgehoben wird. Wie zu erwarten, wird daher die Lichtabsorption der Ketoverbindungen der Formel I bei dar Bildung des Hemiacetales zu kürzeren Wellenlängen, also hypsochrom verschoben.

Bei der praktischen Durchführung wählt man eine Wellenlänge, bei der die Ketoverbindung eine starke Absorption zeigt, und beobachtet das Verschwinden der Absorption bei der angegebenen Wellenlänge aufgrund der Bildung des Hemiacetales mit dem zu bestimmenden Alkohol. Es zeigte sich, dass mit dieser Methode gut reproduzierbare quantitative Bestimmungen des Alkohols möglich sind.

Wie bereits erwähnt wurde, sind prinzipiell Alkohole der Formel

$$R'\text{-}OH ,$$

in welchen R' ein aliphatischer oder cycloaliphatischer Rest ist, zur Bildung der Hemiacetale der Formel II mit den Ketoverbindungen der Formel I geeignet. Bevorzugte Alkohole der angegebenen Formel, die mit den Ketoverbindungen der Formel I Hemiacetale bilden, sind jedoch Alkanole mit 1 - 10 Kohlenstoffatomen. Es zeigte sich ferner, dass primäre Alkohole bei der Bildung des Hemiacetales im Vergleich zu sekundären und tertiären Alkoholen bevorzugt werden.

Bevorzugte Alkoholkomponenten, mit denen die Hemiacetale der Formel II gebildet werden, sind dementsprechend primäre Alkohole mit 1 - 5 Kohlenstoffatomen, und insbesondere Methanol, Aethanol, 1-Propanol und 1-n-Butanol. Entsprechende Tests zeigten, dass die Selektivität der Ketoverbindungen der Formel I gegenüber den primären Alkoholen Methanol, Aethanol, 1-Propanol und 1-n-Butanol sehr ähnlich ist, dass jedoch Isopropanol und tert.Butanol gegenüber den genannten primären Alkoholen um einen Faktor von etwa 10 diskriminiert werden.

Bei der erfindungsgemässen Verwendung der entsprechenden Vorrichtungen zur Bestimmung von Aethanol in wässrigen Systemen, beispielsweise alkoholischen Getränken, ist es nicht störend, dass der festgestellte Aethanolwert durch die Anwesenheit von 1-Propanol oder 1-n-Butanol gestört wird, weil diese Alkoholkomponenten in entsprechenden

Materialien nicht oder nur in vernachlässigbaren Mengen enthalten sind. Methanol kann jedoch in unsauber destillierten Spirituosen in Mengen bis zu 1 g pro 100 ml Aethanol enthalten sein. Somit ist in diesen Fällen eine Störung des bestimmten Aethanolgehaltes aufgrund von Methanol zu erwarten. Es sei in diesem Zusammenhang jedoch darauf hingewiesen, dass auch Aethanolbestimmungen nach den enzymatischen Methoden des Standes der Technik durch die Anwesenheit anderer primärer Alkohole und allenfalls auch sekundärer Alkohole gestört werden.

Alkoholische Getränke, wie Weine, Biere und Liköre, können recht hohe Gehalte an Zuckern aufweisen, und zwar sowohl an Monosacchariden als auch an Disacchariden. Obwohl diese Zuckerbestandteile in ihrem Molekül mehrere Alkoholgruppierungen aufweisen, so sind dennoch die erfindungsgemässe verwendbaren Vorrichtungen bzw Sensoren zur Bestimmung von Alkoholen in solchen alkoholischen Getränken geeignet, die Zuckerbestandteile enthalten, und auch in diesem Falle werden besonders hohe Empfindlichkeiten der Vorrichtungen gegenüber dem Aethanol der alkoholischen Getränke dann erzielt, wenn diese Ketoverbindungen der Formel I, eingebettet in einem Polymermaterial vorliegen. In diesem Falle reagieren ausschliesslich die erwähnten primären Alkohole mit 1 - 5 Kohlenstoffatomen mit den entsprechenden Ketoverbindungen der Formel I, während die in den alkoholischen Getränken vorhandenen Zuckerbestandteile keine Reaktion mit den in das Polymermaterial eingebetteten Ketoverbindungen der Formel I zeigen.

Die Erfindung sei nun anhand von Beispielen erläutert.

Beispiel 1

Herstellung einer Membran zur optischen Bestimmung von Alkoholen in wässrig alkoholischen Lösungen

Die hergestellten Membranen enthielten die Ketoverbindungen der Formel I, eingebettet in einem Polymermaterial, nämlich in einem Polyvinylchlorid eines hohen Molekulargewichts.

Als weitere Komponente enthielten die entsprechenden Polymembranen auf Basis von Polyvinylchlorid einen Weichmacher mit lipophilen Eigenschaften und ausserdem ein quaternäres Ammoniumchlorid, bei welchem an das Stickstoffatom mindestens eine Alkylgruppe mit 4 - 20 Kohlenstoffatomen gebunden ist, und vorzugsweise zwei oder drei langkettige Alkylgruppen mit 8 - 16 Kohlenstoffatomen an dieses Stickstoffatom gebunden sind.

Die hergestellten Membranen wiesen die folgende Zusammensetzung auf:

| Komponente | Gewichtsteile |
|---|---|
| Polyvinylchlorid | 70 - 100 |
| Weichmacher | 50 - 80 |
| quaternäres Ammoniumsalz | 1,5 - 4 |
| Ketoverbindung der Formel I | 5 - 10 |

Zur Herstellung der Membranen zur optischen Bestimmung der Alkohole wurden die oben genannten Komponenten (Polymermaterial, Weichmacher, quaternäres Ammoniumchlorid und Ketoverbindung der Formel I) in einem organischen Lösungsmittel, vorzugsweise frisch destilliertem Tetrahydrofuran, gelöst, und die Lösung auf Platten, beispielsweise Quarzglasplatten, vergossen. Die Lösungen wurden in einer solchen Menge auf die Glasplatte aufgebracht, dass nach dem vollständigen Abdampfen des Lösungsmittels entsprechende Kunststoffmembranen einer Dicke von 3 - 5 µm erhalten wurden, vorzugsweise Kunststoffmembranen einer Dicke von etwa 4 µm.

Bei der Durchführung der Bestimmungen wurden die Membranen jeweils einige Minuten in destilliertem Wasser konditioniert, ehe die Bestimmung des Alkohols in den Messproben durchgeführt wurde.

Beispiel 2

Nach dem in Beispiel 1 beschriebenen Verfahren wurden Membranen zur optischen Bestimmung von Alkoholen hergestellt, welche als Ketoverbindung der Formel I eine solche enthielten, in welcher der Rest Ar ein Benzolkern ist, der als Substituenten eine acylierte Aminogruppe der Formel

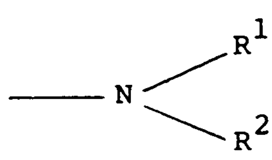

aufweist, in welcher $R^1$ der Acylrest einer Carbonsäure ist und $R^2$ ein Wasserstoffatom oder einen Alkylrest bedeutet, wobei mindestens einer der Reste $R^1$ oder $R^2$ ein längerkettiger Alkylrest, beziehungsweise ein Acylrest einer längerkettigen Alkancarbonsäure, ist, insbesondere entsprechende Alkyl-, beziehungsweise Acylreste mit 8 - 20 Kohlenstoffatomen.

Als Weichmacher wurde in diesen Beispielen ein lipophiler Dicarbonsäurediester, nämlich der Bis(2- äthylhexyl)-sebacinsäureester verwendet.

Als quaternäres Ammoniumchlorid mit lipophilen Eigenschaften wurde gemäss diesen Beispielen das Methyl-tridodecylammonium-chlorid verwendet.

Die Herstellung der Membranen erfolgte nach dem im Beispiel 1 beschriebenen Verfahren, wobei 85 mg Polyvinylchlorid, 65 mg Weichmacher, 2,5 mg des quaternären Ammoniumchlorids, und 7,8 mg einer der hier beschriebenen Ketoverbindungen der Formel I mit acylierter Aminogruppe als Substituent, in 1,5 ml frisch destilliertem Tetrahydrofuran gelöst wurden und die Membranen vergossen wurden, wobei die endgültigen Membranen eine Dicke von etwa 4 µm aufwiesen.

## Beispiel 3

### Durchführung der Bestimmungen

Zur Durchführung der Bestimmung der niederen Alkohole in wässrigen Lösungen wurden die nach dem Verfahren gemäss Beispiel 1, beziehungsweise Beispiel 2, hergestellten Membranen, in den Messzellen verwendet, die in der Veröffentlichung von K. Seiler, W.E. Morf, B. Rusterholz und W. Simon, Anal. Sci., 5 (1989) 557, beschrieben sind. Die Bestimmung wurde in Zellen mit zwei Quarzglasplatten durchgeführt, auf denen die entsprechenden Membranen in der Weise montiert waren, die in der angegebenen Veröffentlichung beschrieben ist, und in der Zelle zu Vergleichszwecken waren auf den Quarzglasplatten keine entsprechenden Membranen montiert.

Bei der Durchführung der Bestimmung wurde die Messzelle und die Zelle zu Vergleichszwecken in ein Spektrophotometer zur Bestimmung der Absorption im ultravioletten Bereich und im sichtbaren Bereich eingeführt und es wurde die Veränderung des Absorptionsspektrums in dem angegebenen Wellenbereich nach der Transmissionsmethode bestimmt. Das verwendete Spektrophotometer war das Modell 555 der Firma Perkin Elmer, Küsnacht, Schweiz.

Um die Temperatur der Messkammer des Spektrophotometers bei der Durchführung der Bestimmungen bei 25°C konstant zu halten, wurde ein speziell entworfener Unterteil der Messzellen mit entsprechend thermostatisiertem Wasser umspült.

Die Eichung erfolgte unter Verwendung von reinem Wasser und Wasser-Alkohol-Mischungen mit Alkoholgehalten bis maximal 65 Vol.-%.

Die getesteten Alkohole waren die folgenden primären Alkohole: Methanol, Aethanol, 1-Propanol und 1-Butanol, sowie ferner tert.-Butanol und Isopropanol.

Die Aktivitätskoeffizienten für Wasser und die verschiedenen Alkohole wurden in den verschiedenen Mischungen berechnet, indem man im Falle der Aethanolbestimmungen den Formalismus von Margules anwandte und im Falle der Bestimmung der anderen genannten Alkohole den Formalismus von Van Laar anwandte, und zwar in derjenigen Weise, wie dies in der Veröffentlichung von J. Gmehling und U. Onken, in Behrens und R. Eckermann (Eds.), Vapor-Liquid Equilibrium Data Collection, Band 1, Verlag und Druckerei Friedrich Bischoff, Frankfurt 1977, beschrieben ist.

Wenn diese Polyvinylchloridmembran, die als Indikator die Ketoverbindung der Formel I enthält, mit einer wässrigen Lösung eines Alkohols der Formel R'OH in Berührung gebracht wird, dann erfolgt eine Extraktion des Alkohols aus der wässrigen Phase in die organische Membranphase, und in der organischen Membranphase wird dann die Ketoverbindung der Formel I durch Reaktion mit dem Alkohol in das Hemiacetal der Formel II umgewandelt.

Da jedoch auch Wasser aus der Wasser und Alkohol enthaltenden Probelösung in die organische Membranphase übertreten kann, und dort mit der Ketoverbindung der Formel I ein entsprechendes Hydrat bilden kann, sind bei dem Kontakt der die Ketoverbindung der Formel I enthaltenden Membran mit der genannten wässrigen Lösung des Alkohols der Formel R'OH die folgenden Gleichgewichte zu erwarten:

$$H_2O(s) + L(org) \underset{}{\overset{K_{H_2O}}{\rightleftharpoons}} L\cdot H_2O(org) \qquad (1)$$

$$R'OH(s) + L(org) \underset{}{\overset{K_{R'OH}}{\rightleftharpoons}} L\cdot R'OH(org) \qquad (2)$$

In den Gleichungen (1) und (2) bedeutet der Index (s) das in der wässrigen Lösung (s für solution) anwesende Wasser, beziehungsweise den in der wässrigen Lösung anwesenden Alkohol R'OH.

L(org) bedeutet den in der Polymermembran, also der organischen Phase, anwesenden elektrisch neutralen Liganden, nämlich die Ketoverbindung der Formel I.

In der Gleichgewichtsreaktion (1) bedeutet der Ausdruck L · $H_2O$(org) die in der Membran durch Reaktion mit dem extrahierten Wasser in die Hydratform umgewandelte Ketoverbindung der Formel I.

In der Reaktionsgleichung (2) bedeutet in analoger Weise der Ausdruck L · R'OH(org) die in der Polymermembran mit dem extrahierten Alkohol in das Hemiacetal der Formel II übergeführte entsprechende Ketoverbindung.

Es hängt von der Gleichgewichtskonstante also

$$K_{H_2O} ,$$

beziehungsweise $K_{R'OH}$ ab, wie weit die Gleichgewichtsreaktion beim Kontakt der Membran mit der sowohl Wasser als auch Alkohol der Formel R'OH enthaltenden wässrigen Lösung in jedem der beiden Fälle (1) und (2) von der linken Seite nach der rechten Seite verschoben wird. Diese Gleichgewichtskonstante hängt wiederum sowohl von der Komplexbildungskonstante für die Bildung des Hydrates, beziehungsweise des Hemiacetales, als auch von dem Verteilungskoeffizient des Wassers einerseits und des Alkohols andererseits zwischen der Probelösung (s) und der organischen Membranphase (org) ab.

Man kann davon ausgehen, dass bei Kontakt der Polymermembran mit den wässrig-alkoholischen Lösungen in der Membranphase ausschliesslich Hydrate der Ketoverbindungen der Formel I und Hemiacetale der Ketoverbindungen der Formel I mit den in der wässrigen Lösung enthaltenen Alkoholen gebildet werden (und überhaupt keine Vollacetale) und somit also ausschliesslich 1:1 Komplexe. Des weiteren kann man davon ausgehen, dass bei unterschiedlichen Konzentrationen der Probelösungen an den jeweiligen Alkohol, die Verteilungskoeffizienten für das Wasser, beziehungsweise den Alkohol, zwischen der Probelösung und der organischen Membranphase konstant bleiben. Unter diesen Voraussetzungen ist der Selektivitätskoeffizient der Optodenmembran für den fraglichen Alkohol, im Vergleich zu dem Wasser durch das Verhältnis der Gleichgewichtskonstanten der beiden betrachteten Spezien gegeben. Der Selektivitätskoeffizient der Optodenmembran für Wasser gegenüber dem jeweils zu bestimmenden Alkohol R'OH wird als

$$K^{opt}_{R'OH,H_2O}$$

bezeichnet, und es gilt dementsprechend für diesen Selektivitätskoeffizienten die folgende Gleichung

$$K^{opt}_{R'OH,H_2O} = K_{H_2O} / K_{R'OH} \quad (3) .$$

Aus dieser Gleichung (3) sieht man also, dass eine entsprechende Optodenmembran einen guten Selektivitätskoeffizienten für den zu bestimmenden Alkohol gegenüber Wasser aufweist, wenn dieser Wert klein ist, also stark unter 1 liegt.

Beispiel 4

Nach dem in Beispiel 3 beschriebenen Verfahren wurde eine Optodenmembran hergestellt, welche als Ketoverbindung der Formel I eine der folgenden Formel

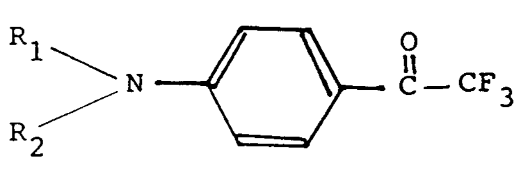

enthielt, wobei in dieser Formel
$R_1$ der Acetylrest

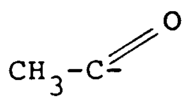

war und

$R_2$ den n-Dodecylrest bedeutete.

Für diese Membran wurden die Gleichgewichtskonstanten für verschiedene Systeme aus Wasser und dem jeweiligen Alkohol bestimmt, und die entsprechenden Selektivitätskoeffizienten berechnet.

In der nachfolgenden Tabelle ist der Logarithmus der Gleichgewichtskonstante K [siehe die vorangegangenen Gleichungen (1) und (2)] für Substrate i angegeben, die Wasser, beziehungsweise die in der Tabelle angeführten Alkohole, sind. Des weiteren ist in dieser Tabelle der Logarithmus des Selektivitätskoeffizienten der jeweiligen Optodenmembran für Aethanol gegenüber Wasser, beziehungsweise gegenüber den anderen in der Tabelle angeführten Alkoholen angegeben, also der Wert für

$$\log K^{opt}_{EtOH,i}$$

angegeben.

Tabelle 1

| Substrat i | $\log K_i$ | $\log K^{opt}_{EtOH,i}$ |
|---|---|---|
| Wasser | 0,2 | -1,1 |
| Aethanol (EtOH) | 1,3 | 0 |
| Methanol | 1,5 | +0,2 |
| 1-Propanol | 1,2 | -0,1 |
| 1-Butanol | 1,5 | +0,2 |
| tert.-Butanol | 0,2 | -1,1 |
| Isopropanol | 0,4 | -0,9 |

Man sieht aus den in der Tabelle angegebenen Werten, dass die entsprechende Optodenmembran eine hohe Selektivität für die Alkohole Methanol, Aethanol, 1-Propanol und 1-Butanol gegenüber Wasser aufweist, und auch eine hohe Selektivität für diese genannten Alkohole gegenüber tert.-Butanol und Isopropanol besitzt.

Die entsprechende Membran ist also gut geeignet um optisch die erwähnten Alkohole in entsprechenden Proben zu bestimmen, welche die jeweiligen Alkohole, gemischt mit Wasser, enthalten.

Aus der oben genannten Tabelle sieht man ferner, dass die entsprechende Membran sogar einen minimal höheren Selektivitätskoeffizienten für Methanol gegenüber Wasser als für Aethanol gegenüber Wasser, aufweist.

Das spezielle Keton der Formel I, das in den hier getesteten Optodenmembranen als optischer Indikator enthalten ist, weist ein Absorptionsmaximum bei 305 nm auf, während das Hemiacetal der Formel II und auch das Hydrat ein Absorptionsmaximum besitzt, das unterhalb von 210 nm ist. Bringt man die entsprechende Optodenmembran mit einer Phosphatpufferlösung eines pH-Wertes von 7 in Berührung, dann bleibt das Absorptionsmaximum bei 305 nm erhalten.

Bringt man die Optodenmembran mit einem Phosphatpuffer in Berührung, der die genannten primären Alkohole Methanol, Aethanol, 1-Propanol und 1-Butanol in steigenden Konzentrationen, beispielsweise im Bereich von 1 Vol.-% bis 33 Vol.-%, enthält, dann verschwindet das Absorptionsmaximum bei 305 nm proportional mit dem Anstieg der Alkoholkonzentration in der getesteten wässrig alkoholischen Probelösung.

Es zeigte sich ferner, dass in einem Diagramm, in welchem die relativen Absorptionswerte bei 305 nm als Funktion des Logarithmuses der Aethanolkonzentration aufgetragen sind, die entsprechende Kurve im Bereich von 1 Vol.-% Aethanol bis 32 Vol.-% Aethanol einen nahezu linearen Verlauf aufweist. Dementsprechend sind die hier beschriebenen Optodenmembranen zur Aethanolbestimmung in alkoholischen Getränken hervorragend gut geeignet.

Beispiel 5

Bestimmung des Alkoholgehaltes in alkoholfreiem Wein, verschiedenen Weissweinen, Rotweinen und Roséweinen, sowie verschiedenen Bieren und gebrannten Getränken unter Verwendung der im Beispiel 4 beschriebenen Optodenmembran

Die Bestimmung wurde nach der im Beispiel 3 beschriebenen Transmissionsmethode in dem dort beschriebenen Geräte bei der Wellenlänge von 305 nm durchgeführt und da die alkoholischen Getränke aufgrund ihrer Färbung selbst bei diesen Wellenlängen eine deutliche Absorption aufweisen können, wurden jeweils 50 ml des zu testenden alkoholischen oder alkoholfreien Getränkes mit 1 g Aktivkohle (das Produkt Purissimum p.a. der Firma Fluka, Buchs, Schweiz) gemischt und anschliessend wurde abfiltriert.

Sämtliche getesteten Proben wurden mit einem Phosphatpuffer eines pH-Wertes von 7 nach dem Abfiltrieren der Aktivkohle im Verhältnis 1:1 verdünnt. Nur im Falle der gebrannten Getränke wurde vor der Zugabe des Puffers noch mit destilliertem Wasser in Verhältnis 2:3 verdünnt, um in den getesteten Proben ähnliche Alkoholkonzentrationen zu erreichen wie beim Wein.

In der nachfolgenden Tabelle 2 sind die Standardabweichungen der mit der Membran bestimmten Werte nach drei Bestimmungen angegeben. Des weiteren wurden in den gleichen Proben die Alkoholwerte auch nach der Destillationsmethode (Destillation der Proben und Bestimmung der Dichte in dem Destillat) bestimmt. Für diese Destillationsmethode wird in der Literatur eine Abweichung von ± 0,1% angegeben (Vol.-% pro Vol.)

Die Alkoholkonzentrationen, die in den angegebenen Proben mit der Optodenmembran und nach der Dichtemethode bestimmt wurden, sind in der nachfolgenden Tabelle angeführt, wobei die Aethanolkonzentrationen und die Standardabweichungen in Vol.-% angeführt sind.

Tabelle 2

| Probe | Optodenmembran | Dichtemethode |
|---|---|---|
| alkoholfreier Wein | 0.71 ± 0.33 | 0.74 |
| Bier | | |
| 1 | 4.95 ± 0.10 | 4.89 |
| 2 | 4.62 ± 0.16 | 4.8 |
| 3 | 5.33 ± 0.14 | 5.4 |
| Weisswein | | |
| 1 | 10.38 ± 0.10 | 10.3 |
| 2 | 10.76 ± 0.07 | 10.9 |
| 3 | 12.05 ± 0.18 | 12.0 |
| Rotwein | | |
| 1 | 12.33 ± 0.04 | 12.4 |
| 2 | 12.90 ± 0.08 | 12.9 |
| 3 | 11.49 ± 0.10 | 11.9 |
| Roséwein | | |
| 1 | 10.58 ± 0.20 | 11.1 |
| 2 | 10.35 ± 0.11 | 10.9 |
| Brände | | |
| 1 | 39.06 ± 0.56 | 39.9 |
| 2 | 39.82 ± 0.19 | 39.8 |
| 3 | 39.84 ± 0.29 | 40.2 |

Man sieht also, dass die mit der Optodenmembran bestimmten Werte bei den getesteten alkoholischen Getränken in sehr guter Uebereinstimmung mit den Werten der Destillations-Dichte-Methode waren. Auch die Standardabweichungen der Bestimmungen der Optodenmembran waren nur geringfügig höher als die aus der Literatur bekannten Standardabweichungen bei der Durchführung der Bestimmung nach dem Destillations-Dichte-Verfahren.

Zieht man die wesentlich raschere Durchführbarkeit der optischen Bestimmung mit den neuen Optodenmembranen in Betracht, dann sieht man den überraschenden Vorteil, der durch dieses Verfahren erzielt wird.

Mit der beschriebenen Membran wurden ferner auch Liköre getestet. Aufgrund des sehr hohen Gehaltes dieser alkoholischen Getränke an Zuckern, war hier die Uebereinstimmung mit den Werten, die nach der Destillations-Dichte-Methode bestimmt wurden, nicht so gut. Die Messungen mit den Optodenmembranen zeigten eine Verfälschung des Messergebnisses in Richtung zu höheren Alkoholgehalten (gegenüber den durch die Destillationsmethode bestimmten Alkoholgehalten von 20 - 30% waren die Werte um 1 - 2 Vol.-% zu hoch). Dies ist aufgrund des hohen Zuckergehaltes der Liköre jedoch nicht überraschend.

## Patentansprüche

1. Verfahren zur Bestimmung von Alkoholen der Formel

R'-OH

in welcher
R' ein aliphatischer oder cycloaliphatischer Rest ist,
dadurch gekennzeichnet, dass man die Alkohole mit einer Ketoverbindung der Formel I

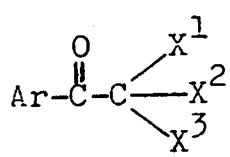

I

in Berührung bringt, in welcher

Ar ein unsubstituierter oder substituierter einkerniger oder mehrkerniger, aromatischer Rest oder ein unsubstituierter oder substituierter ein- oder mehrkerniger heterocyclischer Rest aromatischen Charakters ist und die Substituenten unpolare Substituenten, Substituenten mit schwach polaren Eigenschaften, Substituenten mit basischen Eigenschaften und/oder chromophore Gruppen, beziehungsweise Gruppen, die eine Fluoreszenz oder Lumineszenz induzieren, sind, und

$X^1$, $X^2$ und $X^3$ unabhängig voneinander Wasserstoffatome, Alkylgruppen, Alkenylgruppen, Alkinylgruppen, Fluoratome, Chloratome, Bromatome oder Nitrogruppen bedeuten, unter der Voraussetzung, dass jedoch mindestens einer der Substituenten $X^1$, $X^2$ und $X^3$ ein stark elektronenanziehender Substituent, ausgewählt aus der Gruppe Fluoratome, Chloratome, Bromatome oder Nitrogruppen ist,

wobei sich bei dem Kontakt des Alkohols mit der Ketoverbindung der Formel I reversibel ein Hemiacetal der Formel II

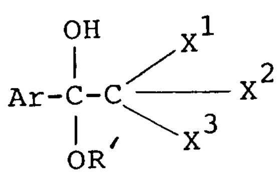

II

bildet und wobei sich die Ketoverbindung der Formel I von dem Hemiacetal bezüglich der Lichtabsorption im ultravioletten, sichtbaren oder infraroten Lichtbereich unterscheidet oder dass bei der Bildung des Hemiacetales eine Fluoreszenz oder Lumineszenz entsteht oder gelöscht wird.

2. Verfahren nach Anspruch 1, dadurch gekennzeichnet, dass man den Alkohol mit einer Ketoverbindung der Formel I in Berührung bringt, in welcher Ar ein Benzolkern, ein Naphthalin-, Phenantren- oder Anthracengerüst ist oder ein ein- oder mehrkerniger heterocyclischer Rest aus der Gruppe Thiophen, Furan, Benzofuran, Benzothiophen, und vorzugsweise ein ein- oder mehrkerniger heterocyclischer Rest aromatischen Charakters ist, der mindestens ein Stickstoffatom als Heteroatom aufweist, vorzugsweise Pyrrol, Diazol, Triazol, Pyridin, Thiazol, 1,2-Diazin, 1,3-Diazin,

1,4-Diazin (Pyrazin), 1,3,5-Triazin, 1,2,4-Triazin oder 1,2,3-Triazin, Tetrazin, Monoazanaphthaline, wie 1-Azanaphthalin (Chinolin) und 2-Azanaphthalin (Isochinolin), Diazanaphthaline, Triazanaphthaline, Indol, Carbazol, Monoazaanthracene, Monoazaphenanthrene, beispielsweise Acridin und Phenanthridin, Diazaanthracene und Diazaphenanthrene, beispielsweise Phenanthrolin, sowie Triaza- und Tetraazaanthracene und -phenanthrene.

**3.** Verfahren nach Anspruch 1 oder 2, dadurch gekennzeichnet, dass in den Ketoverbindungen der Formel I, beziehungsweise den entsprechenden Hemiacetalen der Formel II die an den aromatischen, beziehungsweise heteroaromatischen Rest Ar gebundenen Substituenten 1 - 4, Substituenten ausgewählt sind:

Halogenatome, Nitrogruppen, substituierte oder unsubstituierte Alkylgruppen, substituierte oder unsubstituierte Alkenylgruppen mit ein oder mehr Kohlenstoff-Kohlenstoff-Doppelbindungen, substituierte oder unsubstituierte Alkinylgruppen, substituierte oder unsubstituierte Cycloalkylgruppen, substituierte oder unsubstituierte Arylgruppen, substituierte oder unsubstituierte heterocyclische Reste, wobei die an diesen Gruppierungen allenfalls vorhandenen Substituenten vorzugsweise unpolare oder wenig polare Gruppierungen, wie Halogenatome, Aethergruppierungen, Nitrilgruppen, Carbonsäureestergruppierungen, veresterte Hydroxygruppen, oder basische Gruppierungen, wie primäre, sekundäre oder tertiäre Aminogruppen oder Acylaminogruppen sind,

oder die an die Gruppierung Ar gebundenen Substituenten, Aethergruppierungen, Nitrilgruppen, Carbonsäureestergruppierungen, Hydroxygruppen, veresterte Hydroxygruppen oder primäre, sekundäre oder tertiäre Aminogruppen oder acylierte Aminogruppen der Formel

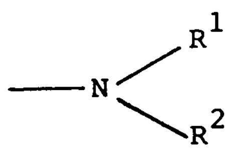

sind, in welcher

die Reste $R^1$ und $R^2$ unabhängig voneinander Wasserstoffatome, substituierte oder unsubstituierte Alkylreste, substituierte oder unsubstituierte Alkenylreste, substituierte oder unsubstituierte Alkinylreste, substituierte oder unsubstituierte Cycloalkylreste, substituierte oder unsubstituierte Arylreste, vorzugsweise Phenylreste, oder substituierte oder unsubstituierte heterocyclische Reste oder Acylgruppierungen der Formeln

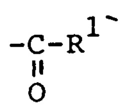

oder

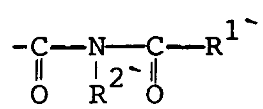

darstellen, in welchen

$R^{1`}$ und $R^{2`}$ die gleiche Bedeutung aufweisen, wie die Reste $R^1$ und $R^2$, ausgenommen die entsprechenden Acylreste, oder

$R^1$ und $R^2$ gemeinsam mit dem Stickstoffatom, an welches sie gebunden sind, einen heterocyclischen Rest bilden, der gegebenenfalls in seiner Ringstruktur noch weitere Heteroatome, ausgewählt aus der Gruppe von Sauerstoff, Schwefel, oder Stickstoff aufweist, und allenfalls Substituenten trägt oder

die Substituenten, die an den aromatischen oder heterocyclischen Rest Ar der Ketoverbindungen der Formel I, beziehungsweise Hemiacetale der Formel II gebunden sind, chromophore Gruppen oder Gruppen, die eine Fluoreszenz oder Lumineszenz induzieren, sind, beispielsweise Azogruppen, über Azogruppen gebundene aromatische oder heteroaromatische Reste oder Systeme mit zwei oder mehr konjugierten Doppelbindungen oder Dreifachbindungen.

4. Verfahren gemäss einem der Ansprüche 1 bis 3, dadurch gekennzeichnet, dass in den Ketoverbindungen der Formel I, beziehungsweise den Hemiacetalen der Formel II, mindestens zwei der Reste $X^1$, $X^2$ und $X^3$ stark elektronenanziehende Substituenten, ausgewählt aus der Gruppe Fluoratome, Chloratome, Bromatome oder Nitrogruppen sind und die Gruppierung der Formel

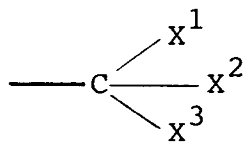

vorzugsweise eine Trifluormethylgruppe ist.

5. Verfahren nach einem der Ansprüche 1 bis 4, dadurch gekennzeichnet, dass man als Ketoverbindung der Formel I eine Azoketoverbindung der Formel III

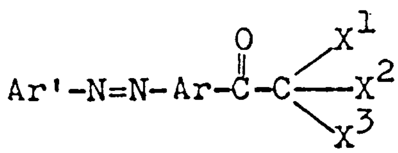

III

verwendet, in welcher die Reste

Ar und Ar' unabhängig voneinander substituierte oder unsubstituierte, ein- oder mehrkernige, aromatische oder substituierte oder unsubstituierte, einoder mehrkernige heterocyclische Reste aromatischen Charakters bedeuten und

$X^1$, $X^2$ und $X^3$ die in Formel I angegebene Bedeutung besitzen.

6. Verfahren gemäss einem der Patentansprüche 1 bis 5, dadurch gekennzeichnet, dass man Alkohole bestimmt in welcher der Rest R' ein Alkyl mit 1 bis 10 Kohlenstoffatomen ist und der entsprechende Alkohol der Formel

R'OH ,

vorzugsweise ein primärer Alkohol mit 1 bis 5 Kohlenstoffatomen und insbesondere Methanol, Aethanol, 1-Propanol oder 1-n-Butanol ist.

7. Verfahren gemäss einem der Ansprüche 1 bis 6, dadurch gekennzeichnet, dass sich die Ketoverbindung der Formel I auf einem oder in einem Trägermaterial befindet, vorzugsweise einem Polymermaterial und dass die Umsetzung der Ketoverbindung der Formel I zu dem Hemiacetal der Formel II auf diesem, beziehungsweise in diesem Trägermaterial stattfindet.

8. Verfahren nach einem der Ansprüche 1-6, dadurch gekennzeichnet, dass die Ketoverbindung der Formel I einen Teil eines optischen Sensors darstellt, der seine Lichtabsorption im ultravioletten, sichtbaren oder infraroten Lichtbereich ändert, oder bei dem eine Fluoreszenz oder Lumineszenz entsteht oder gelöscht wird, sobald dieser optische Sensor mit dem Alkohol der Formel

R'OH

in Berührung kommt und wobei die Ketoverbindung der Formel I den optischen Indikator des Sensors darstellt, der bei Kontakt mit dem zu bestimmenden Alkohol in das Hemiacetal der Formel II umgewandelt wird.

9. Verfahren nach Anspruch 8, dadurch gekennzeichnet, dass der optische Sensor ein Trägermaterial aufweist, in dem oder auf dem sich die Ketoverbindung der Formel I befindet, und dass dieses Trägermaterial vorzugsweise ein Polymermaterial ist, beispielsweise Cellulose, ein Cellulosederivat, ein siliciumenthaltendes Polymermaterial, beispielsweise ein Siliconkautschuk, ein Polymermaterial auf Basis eines Polyesters, eines Polyamides, eines Polyäthers oder ein Homopolymerisat oder Copolymerisat eines äthylenisch ungesättigten Monomers, beispielsweise

ein Homopolymerisat oder Copolymerisat auf Basis von Polystyrol, Polyvinylacetat, Polyvinylalkohol, von Polybutadien, Polyäthylen, Polypropylen und insbesondere eines halogenierten äthylenische ungesättigten Monomers, beispielsweise ein Homopolymerisat oder Copolymerisat von chlorierten oder fluorierten äthylenisch ungesättigten Monomermaterialien, insbesondere Vinylchlorid oder Vinylidenchlorid.

10. Verfahren gemäss Anspruch 9, dadurch gekennzeichnet, dass in der Polymerkomponente des Sensors ein Weichmacher enthalten ist, vorzugsweise ein Weichmacher auf Basis eines Aethers oder eines Esters, wie zum Beispiel eines Dicarbonsäurediesters oder Tetracarbonsäuretetraesters mit jeweils längerkettigen Alkoholkomponenten in diesen Estern, wie zum Beispiel Estern der Adipinsäure oder Sebacinsäure mit Alkoholkomponenten mit 4 bis 12 Kohlenstoffatomen, beispielsweise der Bis(2-aethylhexyl)-sebacinsäureester.

11. Verfahren gemäss Anspruch 9 oder 10, dadurch gekennzeichnet, dass in dem Polymermaterial des Sensors ausserdem eine Komponente enthalten ist, die zu einem Ionenaustausch mit wässrigen Systemen befähigt ist, beispielsweise ein quaternäres Ammoniumsalz, insbesondere ein solches, bei dem mindestens eine Alkylgruppe mit 4 - 20 Kohlenstoffatomen an das Stickstoffatom des quaternären Ammoniumsalzes gebunden ist und, wobei das Anion des quaternären Ammoniumsalzes, beispielsweise ein Halogenidanion, zum Ionenaustausch gegen Ionen der flüssigen oder gasförmigen Probe mit welcher der optische Sensor in Berührung gebracht wird, befähigt ist.

12. Verfahren gemäss einem der Ansprüche 1 bis 11, dadurch gekennzeichnet, dass man eine qualitative, quantitative oder halb-quantitative Bestimmung der Alkohole in flüssigen oder gasförmigen Proben durchführt, beispielsweise in alkoholischen Getränken oder flüssigen oder gasförmigen Proben biologischen Ursprungs, beispielsweise Harn, Blut, Blutserum, Blutplasma oder Atemluft, indem man diese Proben mit einem optischen Sensor in Berührung bringt, der als optischen Indikator eine Ketoverbindung der Formel I enthält.

13. Verwendung einer Vorrichtung, die eine in den Verfahrensansprüchen 1 bis 5 definierte Ketoverbindung der Formel I enthält, zur qualitativen, quantitativen oder halb-quantitativen Bestimmung von Alkoholen der Formel

$$R'OH,$$

in welchen R' ein aliphatischer oder cycloaliphatischer Rest ist, dadurch gekennzeichnet, dass man flüssige oder gasförmige proben, in welchen der Alkoholgehalt bestimmt werden soll, mit der Vorrichtung in Berührung bringt, wobei die in der Vorrichtung als Indikator anwesende Ketoverbindung der Formel I mit dem Alkohol unter Hemiacetalbildung reagiert und dabei eine Aenderung des Absorptionsspektrums hervorgerufen wird, die in ihrer Grössenordnung der Alkoholkonzentration entspricht.

14. Verwendung nach Anspruch 13, dadurch gekennzeichnet, dass man die Vorrichtung zur Bestimmung der Alkoholkonzentration in flüssigen oder gasförmigen Proben, beispielsweise in alkoholischen Getränken oder Proben biologischen Ursprungs, beispielsweise Harn, Blut, Blutserum, Blutplasma oder Atemluft heranzieht.

15. Verwendung gemäss Anspruch 13 oder 14, dadurch gekennzeichnet, dass man eine Vorrichtung verwendet, in welcher sich die Ketoverbindung der Formel I auf einem oder in einem Trägermaterial befindet, vorzugsweise einem Polymermaterial, und dass man diesen Sensor mit der flüssigen oder gasförmigen Probe in Berührung bringt, in welcher der Alkoholgehalt bestimmt werden soll.

## Claims

1. A method of determining alcohols of the formula

$$R'\text{-}OH$$

in which

R' is an aliphatic or cycloaliphatic radical, characterized in that the alcohols are brought into contact with a keto compound of formula I:

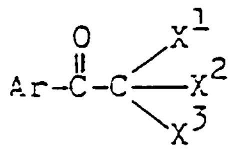

I

in which

Ar is an unsubstituted or substituted, mononuclear or polynuclear aromatic radical or an unsubstituted or substituted, mononuclear or polynuclear heterocyclic radical of aromatic character, the substituents being non-polar substituents, substituents with weakly polar properties, substituents with basic properties and/or chromophoric groups or groups which induce a fluorescence or luminescence, and

$X^1$, $X^2$ and $X^3$ independently of one another are hydrogen atoms, alkyl groups, alkenyl groups, alkynyl groups, fluorine atoms, chlorine atoms, bromine atoms or nitro groups, with the proviso, however, that at least one of the substituents $X^1$, $X^2$ and $X^3$ is a strongly electron-withdrawing substituent selected from the group comprising fluorine atoms, chlorine atoms, bromine atoms or nitro groups,

a hemiacetal of formula II:

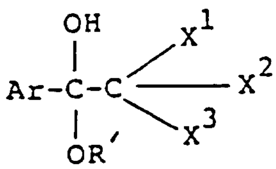

II

being formed reversibly on contact of the alcohol with the keto compound of formula I and the keto compound of formula I differing from the hemiacetal in its light absorption in the ultraviolet, visible or infrared region, or the formation of the hemiacetal being accompanied by the creation or quenching of a fluorescence or luminescence.

**2.** A method according to Claim 1, characterized in that the alcohol is brought into contact with a keto compound of formula I in which Ar is a benzene ring, a naphthalene, phenanthrene or anthracene skeleton or a mononuclear or polynuclear heterocyclic radical from the group comprising thiophene, furan, benzofuran and benzothiophene, and preferably a mononuclear or polynuclear heterocyclic radical of aromatic character which has at least one nitrogen atom as the heteroatom, preferably pyrrole, diazole, triazole, pyridine, thiazole, 1,2-diazine, 1,3-diazine, 1,4-diazine (pyrazine), 1,3,5-triazine, 1,2,4-triazine or 1,2,3-triazine, tetrazine, monoazanaphthalenes such as 1-azanaphthalene (quinoline) and 2-azanaphthalene (isoquinoline), diazanaphthalenes, triazanaphthalenes, indole, carbazole, monoazaanthracenes, monoazaphenanthrenes, for example acridine and phenanthridine, diazaanthracenes and diazaphenanthrenes, for example phenanthroline, and triaza- and tetraaza-anthracenes and -phenanthrenes.

**3.** A method according to Claim 1 or 2, characterized in that, in the keto compounds of formula I or the corresponding hemiacetals of formula II, the substituents bonded to the aromatic or heteroaromatic radical Ar are 1 - 4 substituents selected from:

halogen atoms, nitro groups, substituted or unsubstituted alkyl groups, substituted or unsubstituted alkenyl groups having one or more carbon-carbon double bonds, substituted or unsubstituted alkynyl groups, substituted or unsubstituted cycloalkyl groups, substituted or unsubstituted aryl groups and substituted or unsubstituted heterocyclic radicals, the substituents which may be present in these groupings preferably being non-polar or slightly polar groupings such as halogen atoms, ether groupings, nitrile groups, carboxylic acid ester groupings or esterified hydroxyl groups, or basic groupings such as primary, secondary or tertiary amino groups or acylamino groups,

or the substituents bonded to the grouping Ar are ether groupings, nitrile groups, carboxylic acid ester groupings, hydroxyl groups, esterified hydroxyl groups or primary, secondary or tertiary amino groups or acylated amino groups of the formula

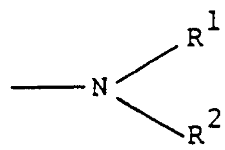

in which
the radicals $R^1$ and $R^2$ independently of one another are hydrogen atoms, substituted or unsubstituted alkyl radicals, substituted or unsubstituted alkenyl radicals, substituted or unsubstituted alkynyl radicals, substituted or unsubstituted cycloalkyl radicals, substituted or unsubstituted aryl radicals, preferably phenyl radicals, or substituted or unsubstituted heterocyclic radicals or acyl groupings of the formula

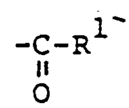

or

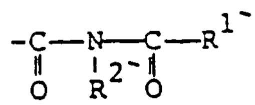

in which
$R^{1'}$ and $R^{2'}$ are defined in the same way as the radicals $R^1$ and $R^2$ with the exception of said acyl radicals, or $R^1$ and $R^2$ form, together with the nitrogen atom to which they are bonded, a heterocyclic radical whose ring structure optionally contains further heteroatoms selected from the group comprising oxygen, sulphur or nitrogen, and which may carry substituents, or
the substituents bonded to the aromatic or heterocyclic radical Ar of the keto compounds of formula I or hemiacetals of formula II are chromophoric groups or groups which induce a fluorescence or luminescence, for example azo groups, aromatic or heteroaromatic radicals bonded *via* azo groups, or systems with two or more conjugated double bonds or triple bonds.

**4.** A method according to one of Claims 1 to 3, characterized in that, in the keto compounds of formula I or hemiacetals of formula II, at least two of the radicals $X^1$, $X^2$ and $X^3$ are strongly electron-withdrawing substituents selected from the group comprising fluorine atoms, chlorine atoms, bromine atoms or nitro groups, and the grouping of the formula

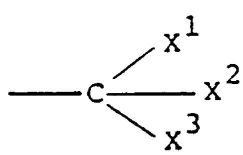

is preferably a trifluoromethyl group.

**5.** A method according to one of Claims 1 to 4, characterized in that the keto compound of formula I used is an azoketo compound of formula III:

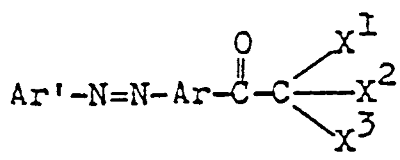 III

in which the radicals

Ar and Ar' independently of one another are substituted or unsubstituted, mononuclear or polynuclear aromatic radicals or substituted or unsubstituted, mononuclear or polynuclear heterocyclic radicals of aromatic character, and

$X^1$, $X^2$ and $X^3$ are as defined in formula I.

**6.** A method according to one of Claims 1 to 5, characterized in that alcohols are determined in which the radical R' is an alkyl having 1 to 10 carbon atoms, and said alcohol of the formula

R'OH

is preferably a primary alcohol having 1 to 5 carbon atoms, especially methanol, ethanol, propan-1-ol or n-butan-1-ol.

**7.** A method according to one of Claims 1 to 6, characterized in that the keto compound of formula I is present on or in a support, preferably a polymer material, and in that the conversion of the keto compound of formula I to the hemiacetal of formula II takes place on or in this support.

**8.** A method according to one of Claims 1 - 6, characterized in that the keto compound of formula I constitutes part of an optical sensor which changes its light absorption in the ultraviolet, visible or infrared region, or in which a fluorescence or luminescence is created or quenched, as soon as this optical sensor comes into contact with the alcohol of the formula

R'OH,

the keto compound of formula I representing the optical indicator of the sensor which is converted to the hemiacetal of formula II on contact with the alcohol to be determined.

**9.** A method according to Claim 8, characterized in that the optical sensor has a support in or on which the keto compound of formula I is present, and in that this support is preferably a polymer material, for example cellulose, a cellulose derivative, a silicon-containing polymer material, for example a silicone rubber, a polymer material based on a polyester, a polyamide or a polyether, or a homopolymer or copolymer of an ethylenically unsaturated monomer, for example a homopolymer or copolymer based on polystyrene, polyvinyl acetate, polyvinyl alcohol, polybutadiene, polyethylene, polypropylene and especially a halogenated, ethylenically unsaturated monomer, for example a homopolymer or copolymer of chlorinated or fluorinated, ethylenically unsaturated monomer materials, especially vinyl chloride or vinylidene chloride.

**10.** A method according to Claim 9, characterized in that the polymer component of the sensor contains a plasticizer; preferably a plasticizer based on an ether or an ester, for example a dicarboxylic acid diester or tetracarboxylic acid tetraester, these esters each having longer-chain alcohol components, for example esters of adipic acid or sebacic acid with alcohol components having 4 to 12 carbon atoms, for example bis(2-ethylhexyl) sebacate.

**11.** A method according to Claim 9 or 10, characterized in that the polymer material of the sensor also contains a component which is capable of ion exchange with aqueous systems, for example a quaternary ammonium salt, especially one in which at least one alkyl group having 4 - 20 carbon atoms is bonded to the nitrogen atom of the quaternary ammonium salt, the anion of the quaternary ammonium salt, for example a halide anion, being capable of ion exchange with ions in the liquid or gaseous sample with which the optical sensor is brought into contact.

**12.** A method according to one of Claims 1 to 11, characterized in that a qualitative, quantitative or semiquantitative determination of alcohols is performed on liquid or gaseous samples, for example on alcoholic drinks or liquid or gaseous samples of biological origin, for example urine, blood, blood serum, blood plasma or respiratory air, by

bringing these samples into contact with an optical sensor which contains a keto compound of formula I as the optical indicator.

**13.** Use of a device containing a keto compound of formula I defined in Process Claims 1 to 5 for the qualitative, quantitative or semiquantitative determination of alcohols of the formula

R'OH

in which R' is an aliphatic or cycloaliphatic radical, characterized in that liquid or gaseous samples whose alcohol content is to be determined are brought into contact with the device, the keto compound of formula I present in the device as the indicator reacting with the alcohol to form a hemiacetal, thereby causing a change in the absorption spectrum which corresponds in its order of magnitude to the alcohol concentration.

**14.** Use according to Claim 13, characterized in that the device is used for determining the alcohol concentration in liquid or gaseous samples, for example in alcoholic drinks or samples of biological origin, for example urea, blood, blood serum, blood plasma or respiratory air.

**15.** Use according to Claim 13 or 14, characterized in that a device is used in which the keto compound of formula I is present on or in a support, preferably a polymer material, and that this sensor is brought into contact with the liquid or gaseous sample whose alcohol content is to be determined.

**Revendications**

**1.** Procédé pour la détermination des alcools de la formule

R'-OH

dont R' est un radical aliphatique ou cycloaliphatique,
caractérisé en ce qu'on met en contact les alcools avec un composé cétonique de la formule I

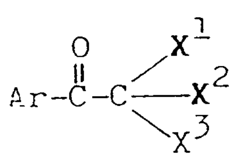

I

dont Ar est radical aromatique substitué ou non-substitué mono- ou multinucléaire ou un radical hétérocyclique de caractère aromatique substitué ou non-substitué mono- ou multinucléaire et les substituants sont des substituants non-polaires, des substituants avec des propriétés polaires faibles, des substituants avec des propriétés basiques et/ou des groupes chromophores, respectivement des groupes induisants une fluorescence ou luminiscence, et

$X^1$, $X^2$ et $X^3$ sont indépendamment des atomes d'hydrogène, des groupes d'alkyle, des groupes d'alcènyle, des groupes d'alcynyle, des atomes de fluor, des atomes de chlore, des atomes de brome ou des groupes de nitro, à condition qu'au moins un de ces substituants $X^1$, $X^2$ et $X^3$ est un substituant fortement attracteur d'électrons sélectionné du groupe comprenant des atomes de fluor, des atomes de chlore, des atomes de brome ou des groupes de nitro,

dont, lors du contact de l'alcool avec le composé cétonique de la formule I, se forme réversiblement un semiacétal de la formule II

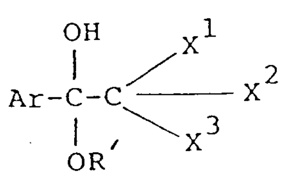

II

et dont le composé cétonique de la formule I se distingue du semi-acétal par son absorption dans la gamme des ultraviolets, visibles ou infrarouges ou par l'apparition ou disparition d'une fluorescence ou luminiscence lors de la formation du semi-acétal.

**2.** Procédé suivant la revendication 1, caractérisé en ce qu'on met en contact l'alcool avec le composé cétonique de la formule I, dans laquelle Ar est un noyau benzène, un système de naphthalène, de phénanthrène ou d'anthracène ou un radical sélectionné du groupe de thiophène, de furane, de benzofurane, de benzothiophène et par préférence un radical hétérocyclique de caractère aromatique mono- ou multinucléaire qui ont au moins un atome d'azote comme hétéroatome, par préférence le pyrrole, le diazole, le triazole, le pyridine, le thiazole, le 1,2-diazine, le 1,3-diazine, le 1,4-diazine (le pyrazine), le 1,3,5-triazine, le 1,2,4-triazine ou le 1,2,3-triazine, le tétrazine, le monoazanaphthlène, comme le 1-azanaphthalène (quinoline) et le 2-azanaphthalène (isoquinoline), le diazanaphthalène, le triazanaphthalène, l'indole, le carbazole, le monoazaanthracène, le monoazaphénanthrène, par exemple l'acridine et le phénanthridine, le diazaanthracène et diazaphénantrène, par exemple phénanthroline, ainsi que des triaza- et tétraazaanthracènes et phénanthrènes.

**3.** Procédé suivant la revendication 1 ou 2, caractérisé en ce que dans les composés cétoniques de la formule I, respectivement les semi-acétals correspondants de la formule II, les substituants 1-4 attachés aux radicaux aromatiques ou hétéro-aromatiques Ar sont des substituants sélectionnés de :

des atomes d'halogène, des groupes de nitro, des groupes d'alkyle substitués ou non-substitués, des groupes d'alcènyle substitués ou non-substitués avec une ou plusieurs doubles-liaisons de carbone-carbone, des groupes d'alcynyle substitués ou non-substitués, des groupes de cycloalkyle substitués ou non-substitués, des groupes d'aryle substitués ou non-substitués, des radicaux hétérocycliques substitués ou non-substitués, dont les substituants sur les groupes sont au besoin des groupes par préférence non-polaires, ou peu polaires, comme des atomes d'halogène, des groupes d'éther, des groupes de nitrile, des groupes d'acide carboxyliques, des groupes hydroxy estérifiés ou des groupes basiques comme des groupes d'amino primaires, secondaires ou tertiaires ou des groupes d'acylamino,

ou que les substituants attachés au groupe Ar sont des groupes d'éther, de nitrile, des groupes d'ester carboxylique, des groupes hydroxy, des groupes hydroxy estérifié ou des groupes d'amino primaires, secondaires ou tertiaires ou des groupes d'amino acylé de la formule

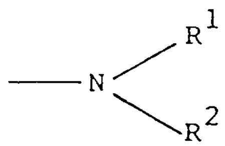

dont les radicaux $R^1$ et $R^2$ représentent indépendamment des atomes d'hydrogène, des radicaux d'alkyle substitués ou non-substitués, des radicaux d'alcènyle substitués ou non-substitués, des radicaux d'alcynyle substitués ou non-substitués, des groupes de cycloalkyle substitués ou non-substitués, des groupes d'aryle substitués ou non-substitués, par préférence des radicaux de phényle, des radicaux hétérocycliques substitués ou non-substitués ou des radicaux d'alcyle de la formule

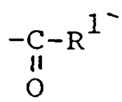

ou

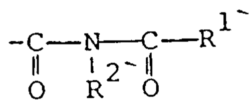

dont $R^{1'}$ et $R^{2'}$ ont la même signification que les radicaux $R^1$ et $R^2$, excepté les radicaux d'alkyle correspondants,

ou $R^1$ et $R^2$ forment ensemble avec l'atome d'azote auquel ils sont liés un anneau hétérocyclique qui a, le cas échéant, d'autres atomes hétéro, sélectionnés du groupe oxygène, soufre ou azote et portent au besoin des substituants ou

les substituants Ar qui sont attachés au radical aromatique ou hétérocyclique du composé cétonique de la formule I, respectivement au semi-acétal de la formule II, sont des groupes chromophores ou des groupes induisants une fluorescence ou luminiscence, par exemple des groupes d'azo, des groupes aromatiques ou hétéroaromatiques qui sont liés à travers des groupes d'azo ou des systèmes avec deux ou plusieurs doubles-liaisons ou triples-liaisons conjuguées.

**4.** Procédé suivant une des revendications 1 à 3, caractérisé en ce que dans les composés cétoniques de la formule I, respectivement les semi-acétals de la formule II, au moins deux des radicaux $X^1$, $X^2$ et $X^3$ sont des substituants fortement attracteurs d'électrons, sélectionnés du groupe des atomes de fluor, des atomes de chlore, des atomes de brome ou des groupes nitro et le groupe de la formule

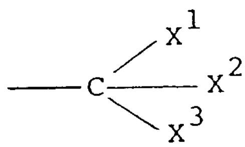

est par préférence un groupe de trifluorméthyle.

**5.** Procédé suivant une des revendications 1 à 4, caractérisé en ce qu'on utilise comme composé cétonique de la formule I un composé azo-cétonique de la formule III

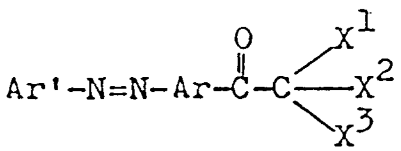 III

dont les radicaux

Ar et Ar' sont indépendamment des radicaux aromatiques substitués ou non-substitués mono- ou multinucléaires ou des radicaux hétérocycliques de caractère aromatique substitués ou non-substitués mono- ou multinucléaire et

$X^1$, $X^2$ et $X^3$ possèdent la signification désignée dans la formule I.

**6.** Procédé suivant une des revendications 1 à 5, caractérisé en ce qu'on détermine des alcools dans lesquels le radical R' est un alkyle avec 1 à 10 atomes de carbone et l'alcool de la formule

R'OH

est par préférence un alcool primaire avec 1 à 5 atomes de carbone et particulièrement du méthanol, de l'éthanol, du 1-propanol ou du 1-n-butanol.

**7.** Procédé suivant une des revendications 1 à 6, caractérisé en ce que le composé cétonique de la formule I se trouve sur un matériel de support, par préférence sur un matériel polymérique et que la réaction du composé cétonique de la formule I conduisant au semi-acétal de la formule II se produit respectivement sur ou dans ledit matériel de support.

**8.** Procédé suivant une des revendications 1 à 6, caractérisé en ce que le composé cétonique de la formule I représente une partie d'un récepteur optique qui change son absorption dans la gamme des ultraviolets, visibles ou infrarouges, ou dont une fluorescence ou luminiscence apparaît ou disparaît dès que ce récepteur entre en contact avec l'alcool de la formule

R'OH

et dont le composé cétonique de la formule I représente l'indicateur du récepteur qui est transformé en semi-acétal de la formule II lors du contact avec l'alcool à déterminer.

9. Procédé suivant la revendication 8, caractérisé en ce que le récepteur optique dispose d'un matériel de support, sur lequel se trouve le composé cétonique de la formule I et que ce matériel de support est par préférence un matériel polymérique, par exemple de la cellulose, un dérivé de cellulose, un matériel polymérique contenant du silicium, par exemple un caoutchouc de silicone, un matériel polymérique basé sur un polyester, un polyamide, un polyéther ou un homopolymère ou un copolymère d'un monomère non-saturé éthylénique, par exemple un homopolymère ou copolymère basé sur du polystyrène, de l'acétate de polyvinyle, de l'alcool de polyvinyle, du polybutadiène, du polyéthylène, du polypropylène et particulèrement d'un monomère non-saturé éthylénique halogéné, par exemple un homopolymère ou copolymère de matériel monomérique non-saturé éthylénique chloré ou fluoré, particulièrement de la chlorure de polyvinyle ou de la chlorure de vinylidène.

10. Procédé suivant la revendication 9, caractérisé en ce que dans la composante polymérique du récepteur, un plastifiant soit contenu, par préférence un plastifiant basé sur un éther ou un ester, comme par exemple un diester dicarboxylique ou tétraacide tétracarboxylique avec des composantes d'alcool avec une longue chaîne dans ces esters, par exemple des esters de l'acide adipique ou l'acide sébacique avec des composantes d'alcool de 4 à 12 atomes de carbone, par exemple l'ester de bis(2-ethylhexyl)-acide sébacique.

11. Procédé suivant les revendications 9 ou 10, caractérisé en ce que dans le matériel polymérique soit, en outre, compris une composante qui a la faculté d'échange d'ions avec des systèmes aqueux, par exemple un sel d'ammonium quaternaire, particulièrement un de ceux auxquels au moins un groupe d'alkyle avec 4-20 atomes de carbone est attaché à l'azote du sel d'ammonium quaternaire et, dont l'anion du sel d'ammonium quaternaire, par exemple un ion d'halogène a la faculté d'échange d'ions contre des ions de l'échantillon liquide ou gazeux, mis en contact avec le récepteur optique.

12. Procédé suivant une des revendications 1 à 11, caractérisé en ce qu'on effectue une détermination qualitative, quantitative ou semi-quantitative des alcools dans des échantillons liquides ou gazeux, par exemple dans des boissons alcoolisées ou des échantillons d'origine biologique, par exemple de l'urine, du sang, du sérum de sang, du plasma du sang ou de l'haleine, en mettant en contact ces échantillons avec un récepteur optique qui contient un composé cétonique de la formule I comme indicateur optique.

13. Utilisation d'un dispositif qui contient un composé cétonique de la formule I défini dans les revendications de procédé 1 à 5 pour la détermination qualitative, quantitative ou semi-quantitative des alcools de la formule

$$R'OH$$

dont R' est un radical aliphatique ou cycloaliphatique, caractérisé en ce qu'on mette en contact des échantillons liquides ou gazeux dont le contenu d'alcool est à déterminer avec le dispositif, alors que le composé cétonique de la formule I qui est présent dans le dispositif comme indicateur, forme avec l'alcool sous formation d'un semiacétal et ainsi suscite un changement du spectre d'absorption qui correspond dans ses proportions à la concentration d'alcool.

14. Utilisation suivant la revendication 13, caractérisée en ce qu'on utilise le dispositif pour la détermination de la concentration d'alcool dans des échantillons liquides ou gazeux, par exemple dans des boissons alcoolisées ou des échantillons d'origine biologique, par exemple de l'urine, du sang, de sérum de sang, du plasma de sang ou de l'haleine.

15. Utilisation suivant les revendications 13 ou 14, caractérisée en ce qu'on utilise un dispositif dans lequel se trouve le composé cétonique de la formule I sur ou dans un matériel de support, par préférence un matériel polymérique et qu'on mette en contact ce récepteur avec l'échantillon liquide ou gazeux, dont le contenu est à déterminer.